(19) Europäisches Patentamt
European Patent Office
Office européen des brevets

(11) **EP 3 654 847 B1**

(12) **FASCICULE DE BREVET EUROPEEN**

(45) Date de publication et mention de la délivrance du brevet:
**18.08.2021 Bulletin 2021/33**

(21) Numéro de dépôt: **18743779.3**

(22) Date de dépôt: **19.07.2018**

(51) Int Cl.:
*A61B 8/08* (2006.01)   *G16H 50/30* (2018.01)

(86) Numéro de dépôt international:
**PCT/EP2018/069688**

(87) Numéro de publication internationale:
**WO 2019/016339 (24.01.2019 Gazette 2019/04)**

(54) **PROCÉDÉ DE CARACTÉRISATION D'UN OS À L'AIDE D'ONDES ULTRASONORES**

VERFAHREN ZUR CHARAKTERISIERUNG VON KNOCHEN MITTELS ULTRASCHALLWELLEN

METHOD FOR CHARACTERISING BONE USING ULTRASONIC WAVES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.07.2017 FR 1756864**

(43) Date de publication de la demande:
**27.05.2020 Bulletin 2020/22**

(73) Titulaires:
• **Sorbonne Université**
  **75006 Paris (FR)**
• **Centre National de la Recherche Scientifique (CNRS)**
  **75016 Paris (FR)**
• **Institut National de la Santé et de la Recherche Médicale (INSERM)**
  **75013 Paris (FR)**
• **Ecole Supérieure de Physique et de Chimie Industrielles de la Ville de Paris**
  **75005 Paris (FR)**

(72) Inventeurs:
• **RENAUD, Guillaume**
  **75015 Paris (FR)**
• **CASSEREAU, Didier**
  **92140 Clamart (FR)**

(74) Mandataire: **Regimbeau**
  **20, rue de Chazelles**
  **75847 Paris Cedex 17 (FR)**

(56) Documents cités:
**US-A- 5 038 787    US-A1- 2005 004 457**

• **NICOLAS BOCHUD ET AL: "Predicting bone strength with ultrasonic guided waves", SCIENTIFIC REPORTS, vol. 7, 3 mars 2017 (2017-03-03), page 43628, XP055467526, DOI: 10.1038/srep43628**

**Description**

**DOMAINE DE L'INVENTION**

La présente invention concerne un procédé de caractérisation d'un os.

**ETAT DE LA TECHNIQUE**

L'utilisation d'ondes pour caractériser un corps vivant est largement connue.

L'imagerie par tomographie à rayons X est par exemple une technique d'imagerie médicale utilisant des rayons X traversant le corps à imager. Cette technique présente toutefois l'inconvénient d'exposer le corps à un rayonnement ionisant potentiellement dangereux. Encore aujourd'hui, on évite d'exposer le corps de jeunes enfants à de tels rayons X sauf absolue nécessité.

D'autres techniques connues ayant pour but de caractériser un corps vivant utilisent des ondes ultrasonores, qui sont des ondes moins dangereuses que les rayons X.

Des ondes ultrasonores sont conventionnellement émises par un réseau d'émetteurs récepteurs, et leurs échos sur un corps à caractériser sont reçus par le même réseau ou un autre réseau après une certaine durée de propagation entre l'émission et la réception.

Des images montrant une coupe du corps dans lequel les ondes ultrasonores se sont propagées peuvent ensuite être construites sur la base des signaux d'écho reçus par le (ou les) réseau(x) d'émetteurs-récepteurs.

Au cours de la construction de telles images, il est fait l'hypothèse que le corps est un milieu homogène, et que, par conséquent, la vitesse du son est uniforme dans le corps étudié.

La vitesse du son choisie est généralement une vitesse du son moyenne dans du tissu biologique non osseux (peau ou muscle par exemple), qui est généralement de l'ordre de 1540 mètres par seconde à une erreur près de 5% à 10%. Les images obtenues sur la base de cette hypothèse présente ainsi une qualité satisfaisante dans des régions d'intérêt montrant du tissu biologique non osseux.

Or, la vitesse du son dans un os (généralement comprise entre 2800 mètres par secondes et 4200 mètres par secondes pour une onde de compression dans l'os cortical) est très supérieure à la vitesse du son dans un tissu biologique non osseux. Des images obtenues sur la base d'une hypothèse de vitesse uniforme dont la valeur correspond à une vitesse du son dans du tissu biologique non-osseux présentent une mauvaise qualité dans des régions d'intérêt montrant l'os. Cette mauvaise qualité se traduit typiquement par une faible intensité et un rendu flou au niveau de l'os. Pour cette raison, une idée reçue largement répandue dans le milieu médical est que les ondes ultrasonores ne « pénètrent pas facilement » dans de l'os.

La différence entre la vitesse du son dans un os et dans les tissus biologiques non osseux entourant l'os crée un effet de réfraction. Il n'est donc pas possible de reconstruire des images de bonne qualité en se fondant sur une valeur prédéterminée de vitesse de son dans l'os, comme on a déjà pu le faire avec une vitesse de son dans un tissu biologique non osseux.

La vitesse du son dans un os dépend de plusieurs facteurs.

Premièrement, la vitesse du son dans un os varie d'un individu à un autre. C'est d'ailleurs la raison pour laquelle elle constitue une information très utile pour diagnostiquer ultérieurement des troubles tels que l'ostéoporose.

Deuxièmement, la vitesse du son dans un os n'est pas la même dans toutes les directions. L'os est en effet un milieu anisotrope. Ceci est dû notamment au fait que l'os dit cortical comprend des canaux s'étendant parallèlement à l'axe longitudinal d'un os long (tibia par exemple), pour héberger des vaisseaux sanguins. Ainsi, une onde sonore de compression se propage dans un os suivant une direction parallèle à l'axe longitudinal d'un os long plus rapidement que suivant une autre direction.

En définitive, déterminer précisément la vitesse de son constitue un enjeu majeur pour caractériser un os.

A cet égard, il a déjà été proposé dans les documents US 2005/004457, US 5 038 787 et « Predicting bone strength with ultrasonc guided waves » des procédés visant à déterminer une vitesse du son dans l'os, ou dans un tissu.

Il a par ailleurs été proposé un procédé de détermination de la vitesse du son dans un os parallèlement à l'axe longitudinal de l'os. Au cours de ce procédé, des ondes ultrasonores sont émises par un réseau d'émetteurs-récepteurs d'ondes ultrasonores alignés parallèlement à l'axe longitudinal d'un os long. Sur la base de signaux d'écho reçus par les récepteurs, on détermine une vitesse d'une onde guidée par l'os, dite onde de tête, se propageant le long de la surface extérieure de l'os. Cette vitesse est déterminée assez facilement en faisant l'hypothèse que la relation entre l'instant de réception d'une onde ultrasonore par un des récepteurs et la distance séparant ce récepteur de l'émetteur de la même onde est une fonction linéaire.

Cependant, ce procédé ne peut pas être utilisé pour déterminer une vitesse du son dans l'os suivant une autre direction qu'une direction parallèle à l'axe longitudinal d'un os long. En conséquence, il ne permet de caractériser un os que de façon partielle.

**EXPOSE DE L'INVENTION**

Un but de l'invention est de proposer un procédé qui puisse être utilisé pour caractériser de façon plus complète un os.

Il est dès lors proposé un procédé de caractérisation d'un os, le procédé comprenant des étapes de :

- émission de premières ondes ultrasonores vers un corps comprenant l'os et du tissu biologique non osseux entourant l'os,
- réception de premiers signaux d'écho des premières ondes ultrasonores émises,
- détermination d'une vitesse du son dans le tissu biologique non osseux suivant une première direction, la détermination comprenant des sous-étapes de :

  ◦ pour plusieurs premières valeurs candidates prédéterminées, construction d'une première image montrant le tissu biologique non osseux et le périoste de l'os, à partir des premiers signaux d'écho et sous l'hypothèse que la vitesse du son dans le tissu biologique non osseux suivant la première direction est égale à la première valeur candidate,
  ◦ pour chaque première image, calcul d'au moins une première métrique indicative d'une qualité de mise au point du périoste et/ou du tissu biologique non osseux entourant l'os dans la première image,
  ◦ sélection d'une des premières valeurs candidates en tant que vitesse du son dans le tissu biologique non osseux suivant la première direction, en fonction des premières métriques,

- localisation d'une première courbe de démarcation entre le tissu biologique non osseux et l'os dans l'une des premières images,
- détermination d'une vitesse du son dans l'os suivant la première direction, la détermination comprenant des sous-étapes de :

  ◦ pour plusieurs deuxièmes valeurs candidates prédéterminées, construction d'une deuxième image montrant du tissu osseux cortical de l'os et l'endoste de l'os, à partir des premiers signaux d'écho, de la vitesse du son dans le tissu biologique non osseux suivant la première direction déterminée, et de la première courbe de démarcation, et sous l'hypothèse que la vitesse du son dans l'os suivant la première direction est égale à la deuxième valeur candidate,
  ◦ pour chaque deuxième image, calcul d'au moins une deuxième métrique indicative d'une qualité de mise au point du tissu osseux cortical de l'os et/ou de l'endoste de l'os dans la deuxième image,
  ◦ sélection d'une des deuxièmes valeurs candidates en tant que vitesse du son dans l'os suivant la première direction, en fonction des deuxièmes métriques.

Le procédé proposé peut également comprendre les caractéristiques ou étapes optionnelles suivantes, prises seules ou en combinaison lorsque cela est techniquement possible.

La construction d'une première image à l'aide d'une première valeur candidate peut comprendre les étapes suivantes mises en œuvre pour au moins un point du tissu biologique non osseux :

- estimation de premières trajectoires de premières ondes ultrasonores émises par des émetteurs, puis passées par le point du tissu biologique non osseux, puis reçues par des récepteurs, à partir des premiers signaux d'écho, sous l'hypothèse que la vitesse du son dans le tissu biologique non osseux suivant la première direction est égale à la première valeur candidate,
- calcul de durées de propagation des ondes ultrasonores via les premières trajectoires estimées,
- calcul d'une intensité d'un pixel de la première image au point du tissu biologique non osseux, à partir des durées de propagation, des premiers signaux d'écho et de positions des émetteurs et des récepteurs.

La construction d'une deuxième image à l'aide d'une deuxième valeur candidate comprend les étapes suivantes mises en œuvre pour au moins un point de l'os :

- estimation de deuxièmes trajectoires de premières ondes ultrasonores émises par des émetteurs, puis passées par le point de l'os, puis reçues par des récepteurs, à partir des signaux d'écho, à partir de la vitesse du son dans le tissu biologique non osseux suivant la première direction déterminée, de la première courbe de démarcation, et sous l'hypothèse que la vitesse du son dans l'os suivant la première direction est égale à la deuxième valeur candidate,
- calcul de durées de propagation des ondes ultrasonores via les deuxièmes trajectoires estimées,

- calcul d'une intensité d'un pixel de la deuxième image au point de l'os, à partir des durées de propagation, des premiers signaux d'écho et de positions des émetteurs et des récepteurs.

La localisation de la première courbe de démarcation peut être mise en œuvre dans la première image qui a été construite à l'aide de la première valeur sélectionnée en tant que vitesse du son dans le tissu biologique non osseux suivant la première direction.

Les premières ondes ultrasonores peuvent être des ondes émises par des émetteurs et les signaux d'échos reçus par des récepteurs alignés le long d'un axe perpendiculaire à un axe longitudinal de l'os, et dans lequel la première direction est une direction perpendiculaire à l'axe longitudinal de l'os.

Le procédé peut comprendre un affichage de la première image construite à l'aide de la première valeur sélectionnée, et/ou de la deuxième image construite à l'aide de la deuxième valeur sélectionnée.

Le procédé peut en outre comprendre des étapes de :

- émission de deuxièmes ondes ultrasonores vers le corps,
- réception de signaux d'écho des deuxièmes ondes ultrasonores émises, dits deuxièmes signaux d'écho,
- détermination d'une vitesse du son dans le tissu biologique non osseux suivant une deuxième direction différente de la première direction, comprenant des sous-étapes de :

  ∘ pour plusieurs troisièmes valeurs candidates, construction d'une troisième image montrant le tissu biologique non osseux et le périoste de l'os, à partir des deuxièmes signaux d'écho et sous l'hypothèse que la vitesse du son dans le tissu biologique non osseux suivant la deuxième direction est égale à la deuxième valeur candidate,
  ∘ pour chaque troisième image, calcul d'au moins une troisième métrique indicative d'une qualité de mise au point du périoste et/ou du tissu biologique non osseux dans la troisième image,
  ∘ sélection d'une des troisièmes valeurs candidates en tant que vitesse du son dans le tissu biologique non osseux suivant la deuxième direction, en fonction des troisièmes métriques,

- localisation d'une deuxième courbe de démarcation entre le tissu biologique non osseux et le périoste dans l'une des troisièmes images,
- détermination d'une vitesse du son dans l'os suivant la deuxième direction, à l'aide des deuxièmes signaux d'écho,
- détermination d'un paramètre d'anisotropie de l'os susceptible d'être utilisé par une fonction prédéterminée en combinaison avec la vitesse du son dans l'os suivant la première direction et avec la vitesse du son dans l'os suivant la deuxième direction pour calculer une vitesse du son dans l'os suivant une direction quelconque, la détermination du paramètre d'anisotropie comprenant des sous-étapes de :

  ∘ pour plusieurs quatrièmes valeurs candidates prédéterminées, construction d'une quatrième image montrant du tissu osseux cortical et l'endoste de l'os, à partir des deuxièmes signaux d'écho, de la vitesse de son dans l'os suivant la première direction, de la deuxième courbe de démarcation, de la vitesse du son dans le tissu biologique non osseux suivant la deuxième direction, et éventuellement de la vitesse de son dans l'os suivant la deuxième direction, de la fonction prédéterminée, et sous l'hypothèse que le paramètre d'anisotropie de l'os est égal à la quatrième valeur candidate,
  ∘ pour chaque quatrième image, calcul d'une quatrième métrique indicative d'une qualité de mise au point de l'endoste et/ou du tissu osseux cortical de l'os dans la quatrième image,
  ∘ sélection d'une des quatrièmes valeurs candidates en tant que paramètre d'anisotropie de l'os, en fonction des quatrièmes métriques.

La construction d'une quatrième image peut comprendre les étapes suivantes mises en œuvre pour au moins un point de l'os :

- estimation de troisièmes trajectoires de deuxièmes ondes ultrasonores émises par des émetteurs, puis passées par le point de l'os, puis reçues par des récepteurs, à partir des deuxièmes signaux d'écho, de la vitesse du son dans le tissu biologique non osseux, et éventuellement de la vitesse de son dans l'os suivant la première direction déterminée, de la vitesse du son dans l'os suivant la deuxième direction déterminée, de la deuxième courbe de démarcation, de la fonction prédéterminée et sous l'hypothèse que le paramètre d'anisotropie de l'os est égale à la quatrième valeur candidate,
- calcul de durées de propagation des ondes ultrasonores via les troisièmes trajectoires estimées,
- calcul d'une intensité d'un pixel de la quatrième image au point de l'os, à partir des durées de propagation et des deuxièmes signaux d'écho et de positions des émetteurs et des récepteurs.

La localisation de la deuxième courbe de démarcation peut être mise en œuvre dans la troisième image ayant été construite à l'aide de la troisième valeur sélectionnée en tant que vitesse du son dans le tissu biologique non osseux suivant la deuxième direction.

Le procédé peut comprendre un affichage de la troisième image construite à l'aide de la troisième valeur sélectionnée, et/ou de la quatrième image construite à l'aide de la quatrième valeur sélectionnée et de la vitesse du son dans l'os suivant la deuxième direction.

Les deuxièmes ondes ultrasonores peuvent être des ondes émises par des émetteurs et les deuxièmes signaux d'écho sont reçus par des récepteurs alignés le long d'un axe compris dans un plan qui comprend par ailleurs un axe longitudinal de l'os, et dans lequel la deuxième direction est de préférence une direction parallèle à l'axe longitudinal de l'os.

La ou chaque vitesse de son dans l'os déterminée peut être une vitesse de propagation d'ondes de compression ou une vitesse de propagation d'ondes de cisaillement.

La ou chaque vitesse de son dans l'os déterminée est une vitesse de propagation d'ondes de compression, et la fonction prédéterminée être une fonction V de la forme :

$$V(\theta) = V_{os2} - (V_{os2} - V_{os1})[\beta \sin^2(\theta) \cos^2(\theta) + \cos^4(\theta)]$$

dans lequel

- $V_{os1}$ est la vitesse de propagation d'ondes de compression dans l'os suivant la première direction déterminée,
- $V_{os2}$ est la vitesse de propagation d'ondes de compression dans l'os suivant la deuxième direction déterminée,
- $\beta$ est le paramètre d'anisotropie de l'os,
- $\theta$ est un angle,
- $V(\theta)$ est une vitesse de propagation d'ondes de compression dans l'os suivant une direction formant l'angle $\theta$ avec la première direction.

La vitesse de son dans l'os suivant la première direction déterminée peut être une vitesse de propagation d'ondes de cisaillement, et la fonction prédéterminée être une fonction V de la forme :

$$V(\theta) = V_{os1}[1 + \beta \sin^2(\theta) \cos^2(\theta)]$$

dans lequel

- $V_{os1}$ est la vitesse de propagation d'ondes de cisaillement dans l'os suivant la première direction déterminée,
- $\beta$ est le paramètre d'anisotropie de l'os,
- $\theta$ est un angle,
- $V(\theta)$ est une vitesse de propagation d'ondes de cisaillement dans l'os suivant une direction formant l'angle $\theta$ avec la première direction.

Il peut être déterminé, conjointement à la vitesse du son dans l'os suivant la première direction, un paramètre d'anisotropie de l'os susceptible d'être utilisé par une fonction prédéterminée en combinaison avec la vitesse du son dans l'os suivant la première direction déterminée pour calculer une vitesse du son dans l'os suivant une direction quelconque, ladite détermination conjointe comprenant des sous-étapes de :

- pour plusieurs paires de deuxièmes et quatrièmes valeurs candidates prédéterminées, construction d'une deuxième image montrant du tissu osseux cortical de l'os et l'endoste de l'os, à partir des premiers signaux d'écho, de la vitesse du son dans le tissu biologique non osseux suivant la première direction déterminée, et de la première courbe de démarcation, sous l'hypothèse que la vitesse du son dans l'os suivant la première direction est égale à la deuxième valeur candidate, et sous l'hypothèse que le paramètre d'anisotropie de l'os est égal à la quatrième valeur candidate,
- pour chaque deuxième image, calcul d'au moins une deuxième métrique indicative d'une qualité de mise au point du tissu osseux cortical de l'os et/ou de l'endoste de l'os dans la deuxième image,
- sélection conjointe d'une deuxième valeur candidate en tant que vitesse du son dans l'os suivant la première direction, et d'une quatrième valeur candidate en tant que paramètre d'anisotropie de l'os, la sélection étant mise en œuvre en fonction des deuxièmes métriques.

**DESCRIPTION DES FIGURES**

D'autres caractéristiques, buts et avantages de l'invention ressortiront de la description qui suit, qui est purement illustrative et non limitative, et qui doit être lue en regard des dessins annexés sur lesquels :

- La figure 1 est une vue schématique en perspective d'un corps comprenant de l'os.
- La figure 2 est une représentation schématique d'un système de caractérisation d'un os, selon un mode de réalisation de l'invention.
- La figure 3 montre certaines étapes d'un procédé de caractérisation d'un os, selon un premier mode de réalisation de l'invention.
- Les figures 4 et 6 détaillent des sous-étapes d'étapes du procédé selon le premier mode de réalisation de l'invention.
- Les figures 5a et 5b sont deux exemples d'images construites au cours de la mise en œuvre du procédé selon le premier mode de réalisation de l'invention.
- La figure 7 est un plan de coupe longitudinal d'un corps comprenant de l'os, montrant également le trajet d'une onde ultrasonore dans ce corps.
- La figure 8 est un ensemble de courbes, chaque courbe montrant l'évolution d'une métrique calculée au cours de la mise en œuvre du procédé selon le premier mode de réalisation de l'invention, en fonction d'une valeur candidate de vitesse de propagation d'ondes de compression radiales dans un os.
- La figure 9 est montre d'autres étapes du procédé de caractérisation d'un os selon le premier mode de réalisation de l'invention.
- Les figures 10 et 11 détaillent des sous-étapes d'étapes représentées en figure 9.
- La figure 12 est un ensemble de courbes, chaque courbe montrant l'évolution d'une métrique calculée lors de la mise en œuvre du procédé selon le premier mode de réalisation de l'invention, en fonction d'une valeur candidate pour un paramètre d'anisotropie d'un os.
- La figure 13 est un organigramme d'étapes d'un procédé de caractérisation d'un os, selon un deuxième mode de réalisation de l'invention.
- La figure 14 détaille des sous-étapes d'une étape du procédé selon le deuxième mode de réalisation de l'invention.

Sur l'ensemble des figures, les éléments similaires portent des références identiques.

**DESCRIPTION DETAILLEE DE MODES DE REALISATION DE L'INVENTION**

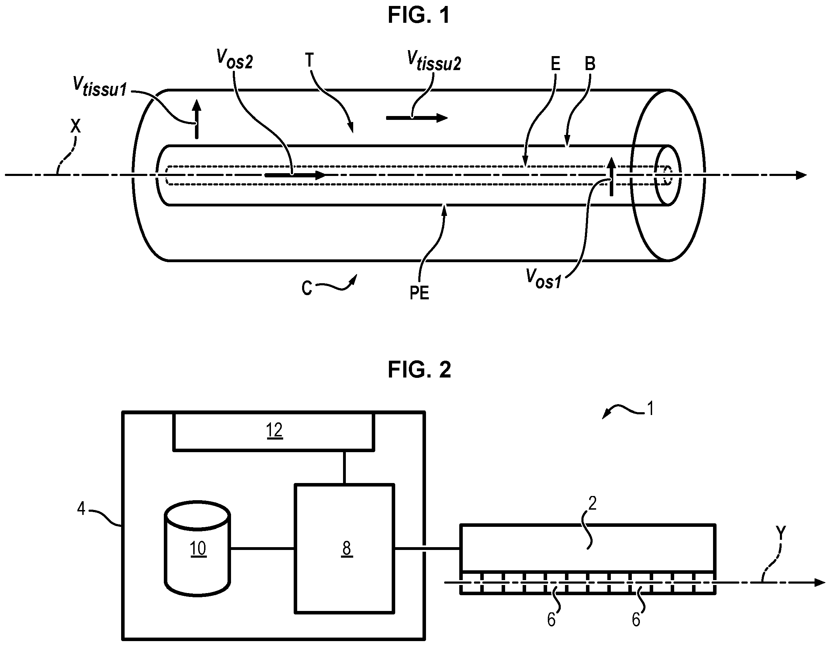

On a illustré de manière schématique en **figure 1** un exemple de partie d'un corps C comprenant un os B et du tissu biologique non osseux T.

L'os B s'étend le long d'un axe longitudinal X. L'os B comprend notamment de la moelle, un endoste E s'étendant autour de la moelle (représenté en pointillés sur la figure 1), et un périoste PE qui entoure l'endoste E. Le périoste PE constitue une couche extérieure de l'os B. L'os B comprend par ailleurs du tissu osseux cortical se trouvant entre l'endoste E et le périoste PE.

Le tissu biologique non osseux T s'étend autour de l'os, et plus spécifiquement autour du périoste PE avec lequel il est en contact.

Le tissu biologique non osseux T comprend de la chair voire de la peau entourant la chair.

De façon bien connue, des ondes ultrasonores peuvent se propager dans un tel corps C,

A cet égard, on a représenté en figure 1 plusieurs vitesses propagation d'ondes sur la figure 1, qui sont les suivantes :

- $V_{os1}$ : vitesse de propagation d'une onde de compression dans l'os B suivant une direction perpendiculaire à l'axe longitudinal X de l'os B (on parlera alors de « direction radiale », et d'ondes de compression « radiales »),
- $V_{os2}$ : vitesse de propagation d'une onde de compression dans l'os B suivant une direction parallèle à l'axe longitudinal X (on parlera alors de direction « axiale » et d'ondes de compression « axiales »),
- $V_{tissu1}$ : vitesse de propagation d'une onde de compression dans le tissu biologique non osseux T suivant une direction perpendiculaire à un axe longitudinal d'un os,
- $V_{tissu2}$ : vitesse de propagation d'une onde de compression dans le tissu biologique non osseux T suivant une direction parallèle à l'axe longitudinal X de l'os B.

La propagation d'ondes de compression dans un os suivant une direction quelconque peut être calculée au moyen d'une fonction prédéterminée V combinant un paramètre d'anisotrope $\beta$ et des vitesses $V_{os2}$ et $V_{os1}$.

La fonction prédéterminée V est alors typiquement de la forme suivante :

$$V(\theta) = V_{os2} - (V_{os2} - V_{os1})[\beta \sin^2(\theta) \cos^2(\theta) + \cos^4(\theta)]$$

où

- $\theta$ est un angle quelconque,
- $\beta$ est un paramètre d'anisotropie de l'os,
- $V(\theta)$ est une vitesse du son dans l'os B suivant une direction formant l'angle $\theta$ avec la première direction.

Cette fonction V se fonde sur l'hypothèse relativement réaliste que l'os B est isotrope transverse, c'est-à-dire isotrope dans un plan perpendiculaire à son axe longitudinal X.

En référence à la **figure 2,** un système 1 de caractérisation comprend une sonde 2 ultrasonore, un dispositif de traitement 4 de signaux d'écho acquis par la sonde 2, voire un écran d'affichage 12.

La sonde 2 ultrasonore, connue en elle-même, comprend au moins un réseau d'émetteurs-récepteurs 6 alignés le long d'un axe Y.

De façon conventionnelle, la sonde 2 comprend une lentille en silicone (non illustrée) agencée devant la rangée d'émetteurs-récepteurs 6.

Chaque émetteur-récepteur 6 est adapté pour émettre des ondes ultrasonores.

Chaque émetteur-récepteur 6 est également adapté pour acquérir des signaux d'écho d'ondes ultrasonores émises par un quelconque autre émetteur-récepteur 6.

Les positions relatives des émetteurs-récepteurs 6 sont prédéterminées. Typiquement, les émetteurs-récepteurs 6 sont distants d'un pas constant le long de l'axe de la sonde 2.

Par ailleurs, le dispositif de traitement 4 de signaux d'écho comprend conventionnellement au moins un processeur 8 et une mémoire 10.

Le processeur 8 est configuré pour exécuter des calculs, et en particulier un algorithme de traitement d'images dont le fonctionnement sera détaillé dans la suite.

La mémoire 10 stocke des données prédéterminées. Ces données ne sont pas propres au corps d'un individu mais sont des données génériques applicables à tout individu d'une population.

Les données prédéterminées comprennent un ensemble de premières valeurs candidates prédéterminées pour la vitesse $V_{tissu1}$. Les premières valeurs candidates prédéterminées sont typiquement comprises entre 1400 et 1700 mètres par seconde.

Les données prédéterminées comprennent par ailleurs un ensemble de deuxièmes valeurs candidates de vitesse pour la vitesse $V_{os1}$. Les deuxièmes valeurs candidates prédéterminées sont typiquement comprises entre 2600 et 3600 mètres par seconde.

Les données prédéterminées comprennent un ensemble de troisièmes valeurs candidates prédéterminées pour la vitesse $V_{tissu2}$. Les troisièmes valeurs candidates prédéterminées sont typiquement comprises entre 1400 et 1700 mètres par seconde.

Les données prédéterminées comprennent par ailleurs un ensemble de quatrièmes valeurs candidates pour le paramètre $\beta$ d'anisotropie de l'os B. Les quatrièmes valeurs sont typiquement comprises entre 0.8 et 2.

Comme on le verra dans la suite, l'algorithme mis en oeuvre par le processeur 8 fait la supposition que la vitesse de propagation dans un os B est régie par la fonction V décrite plus haut, laquelle dépend notamment du paramètre $\beta$ d'anisotropie.

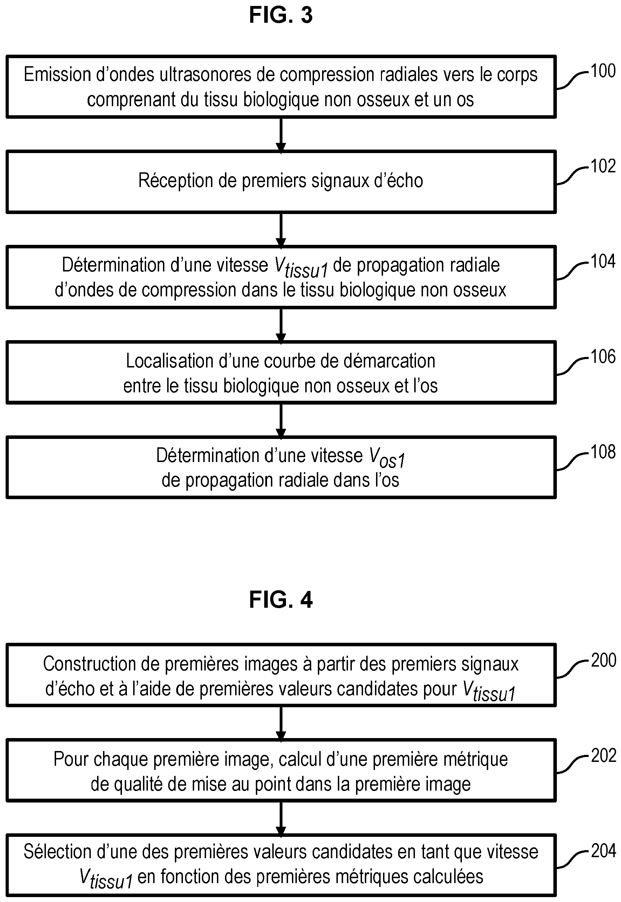

En référence à l'organigramme de la **figure 3,** un procédé de caractérisation de l'os B représenté en figure 1 au moyen du système de caractérisation 1 comprend les étapes suivantes.

La sonde 2 est positionnée à proximité du corps C dans une première position.

Dans la première position, les émetteurs-récepteurs 6 de la sonde 2 sont alignés sensiblement perpendiculairement à l'axe longitudinal de l'os B. Autrement dit l'axe Y de la sonde 2 est perpendiculaire à l'axe X de l'os B dans la première position.

La sonde 2 émet des premières ondes ultrasonores en direction du corps C suivant une première direction (étape 100).

Les premières ondes sont par exemple des ondes de compression radiales. Autrement dit, la première direction est une direction perpendiculaire à l'axe longitudinal de l'os B.

Les premières ondes ultrasonores pénètrent dans le corps C et sont réfléchies par ce dernier à différents niveaux de profondeur dans le corps C.

Des signaux d'écho de ces ondes, dits premiers signaux d'écho, sont ainsi acquis par les émetteurs récepteurs de la sonde 2 (étape 102). Bien entendu, une onde émise par un émetteur-récepteur 6 d'indice i peut parfaitement donner naissance à un signal d'écho reçu par un autre émetteur-récepteur 6 d'indice j.

Les premiers signaux d'écho sont numérisés, transmis au dispositif de traitement 4 et mémorisés dans la mémoire 10 sous une forme connue de l'état de la technique.

Le processeur 8 détermine la vitesse $V_{tissu1}$ sur la base des premiers signaux d'écho reçus et sur la base des premières valeurs prédéterminées mémorisées dans la mémoire 10 (étape 104).

En référence à la **figure 4,** la détermination 104 de la vitesse $V_{tissu1}$ comprend les sous-étapes suivantes.

Le processeur 8 construit une image, dite première image, à partir des premiers signaux d'écho, sous l'hypothèse que la vitesse $V_{tissu1}$ est égale à une première valeur candidate (étape 200).

La première image est constituée d'une grille de pixels, chaque pixel étant défini par une position dans la grille et par une intensité I, cette intensité étant typiquement représentative d'un niveau de gris. La première image représente par ailleurs une vue en coupe du corps C dans un plan dans lequel les premières ondes ultrasonores se sont propagées (ce plan de coupe étant parallèle à l'axe de la sonde 2). Chaque point de ce plan de coupe sera ainsi montré dans un pixel de la première image.

Dans une variante de réalisation préférée, la construction de la première image est réalisée au moyen de la méthode de migration de Kirchhoff ou de la méthode dite « de mise au point totale » (« Total Focusing Method » en anglais). Ces méthodes sont connues en elles-mêmes, mais avantageusement appliquées aux premiers signaux ultrasonores que l'on a précédemment acquis.

La construction 200 de la première image au moyen d'une de ces méthodes connues comprend les sous-étapes suivantes.

Pour un point P donné du corps C étudié, le processeur 8 estime les trajectoires de premières ondes ultrasonores émises par les émetteurs, puis passées par le point P, puis reçues par des récepteurs, à partir des premiers signaux d'écho, sous l'hypothèse que la vitesse $V_{tissu1}$ est égale à une des première valeur candidate.

Les ondes ultrasonores passées par le point P ont été chacune émise par un émetteur d'indice i, dont la position est connue le long de l'axe Y de la sonde 2, et reçue par un récepteur d'indice j, dont la position est également connue le long de l'axe Y de la sonde 2. On a donc au plus autant de premiers signaux d'écho d'ondes passées par le point P que de paires (i, j) d'indices d'émetteurs/récepteurs dans la sonde 2 (donc au plus M x N signaux si M est le nombre d'émetteurs utilisés et N le nombre de récepteurs utilisés).

L'estimation des trajectoires des ondes ultrasonores est mise en œuvre en exploitant le principe de Fermât, selon lequel il est supposé qu'une onde se propage rectilignement dans un milieu homogène. Ici, le corps C comprenant du tissu biologique non osseux et de l'os B est considéré dans son ensemble comme un milieu homogène. Il est fait l'hypothèse que la vitesse de propagation d'une onde est égale à la première valeur candidate considérée, qui correspond à une valeur crédible de vitesse de propagation du son dans du tissu biologique non osseux T.

Dans une autre variante de réalisation, l'étape de construction 200 de la première image est mise en œuvre au moyen de la méthode dite de migration par renversement temporel (« Reverse Time Migration » en anglais, abrégé en RTM). Cette méthode est une méthode d'imagerie alternative aboutissant à une image représentant la réflectivité d'une région en tout point de celle-ci. Elle suppose la connaissance de la géométrie du milieu étudié et de la vitesse de propagation des ondes en chaque point. L'image de réflectivité est obtenue en calculant, en tout point de l'image, une corrélation temporelle entre un champ incident généré par un émetteur et le champ rétro-propagé enregistré par les récepteurs. Ces champs sont obtenus en résolvant numériquement l'équation des ondes acoustiques (ou élastiques), en utilisant respectivement la forme d'onde générée par un émetteur et les signaux d'écho enregistrés par les récepteurs (renversés dans le temps) comme conditions aux limites. Ces opérations doivent être répétées pour chaque émission. L'image finale est obtenue en sommant les images obtenues pour chaque émission. Cette méthode est néanmoins beaucoup plus coûteuse en temps de calcul que celle utilisée dans la variante de réalisation préférée décrite précédemment.

Le processeur 8 calcule ensuite des durées de propagation des ondes passées par le point P via les trajectoires estimées.

Une durée de propagation se décompose en une durée de propagation $t_T(i,P)$ depuis l'émetteur d'indice i jusqu'au point P, et une durée de propagation $t_R(j,P)$ depuis le point P jusqu'au récepteur d'indice j.

Le processeur 8 calcule ensuite une intensité d'un pixel de la première image au point P considéré, à partir des durées de propagation estimées, des premiers signaux d'écho et des positions des émetteurs et des récepteurs.

L'intensité I du point P est typiquement calculée via la formule ci-dessous :

$$I(P) = \sum_{i=1}^{M} \sum_{j=1}^{N} W(P,i,j) \times D(t = t_T(i,P) + t_R(j,P), i, j)$$

dans laquelle :

- $D(t = t_T(i, P) + t_R(j, P), i,j))$ désigne une donnée représentative d'un signal d'écho reçu à l'instant t par le récepteur d'indice j, l'écho provenant d'une onde initialement émise par l'émetteur d'indice i,

- $W(P, i,j)$ désigne un poids obtenu par application d'une fonction de pondération W prédéterminée.

Typiquement, la fonction de pondération W est une fonction fenêtre d'observation (également appelée fenêtre de pondération ou d'apodisation dans la littérature). On a $W(P, i,j) = 1$ si l'angle du segment de trajet retour de l'onde allant du point P au récepteur d'indice j, par rapport à une direction normale à un plan d'émission/réception de la sonde 2, est inférieur à un seuil angulaire prédéterminé, et l'on a $W(P, i,j) = 0$ sinon. Ce seuil angulaire est par exemple fixé à 50 degrés (cet angle correspondant à une perte de sensibilité d'un récepteur de l'ordre de 50%).

En répétant les sous étapes qui précèdent en plusieurs points P, on peut reconstruire une première image. L'étape de construction 200 d'une première image est alors terminée.

L'étape de construction 200 d'une première image est répétée pour chaque première valeurs mémorisée dans la mémoire 10.

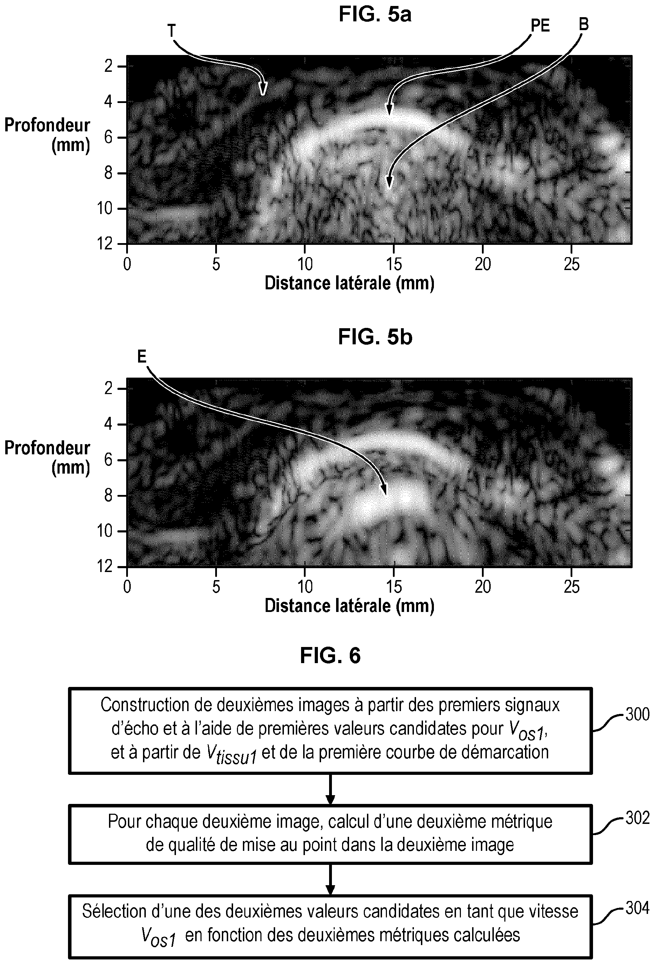

On a représenté en **figure 5a** un exemple de première image que l'on peut obtenir par la mise en œuvre de l'étape de construction 200. Comme on peut le constater, cette première image est relativement riche en information au niveau du tissu biologique non osseux T entourant l'os. En revanche, cette première image est pauvre en information à l'intérieur de l'os. La première image présente notamment une zone floue au niveau de l'os B. Ceci est dû au fait que le processeur 8 a pris comme vitesse de propagation de référence une première valeur candidate qui correspond à une valeur crédible de vitesse de propagation du son dans du tissu biologique non osseux T, mais qui ne correspond pas à une valeur crédible de vitesse de propagation du son dans un os, comme cela a été évoqué dans l'introduction du présent texte.

De retour à la figure 4, pour une première image construite au cours de l'étape 200, le processeur 8 calcule une première métrique représentative d'une qualité de mise au point dans une région d'intérêt de la première image (étape 202). La région d'intérêt choisie est typiquement une région montrant le périoste PE et/ou du tissu biologique non osseux T entourant l'os B.

La première métrique est de préférence fonction d'une intensité moyenne et/ou d'un contraste moyen dans la région d'intérêt considérée dans la première image.

La première métrique est typiquement l'une ou une combinaison des métriques suivantes, connues de l'état de la technique :

- La métrique d'énergie spectrale latérale décrite dans le document « Sound Speed Correction in ultrasound image » par D. Napolitano, C.Chou, G. McLaughlin et al., publié en 2006,
- la métrique dite de « critère de netteté par la méthode de Brenner » ou la métrique dite de « critère de netteté par la méthode de Tenenbaum » ou la métrique appelée « critère de netteté par la méthode de la variance », toutes décrites dans le document « Automatic sound speed selection in photoacoustic image reconstruction using an autofocus approach », par B. Treeby, T. Varslot, E. Zhang et al., publié en 2011.

Le processeur 8 répète l'étape 202 pour chaque première image construite au cours de l'étape 200. Le processeur 8 génère ainsi autant de premières métriques que de premières images, et que de premières valeurs candidates prédéterminées.

Le processeur 8 sélectionne ensuite en tant que valeur définitive pour la vitesse $V_{tissu1}$ une valeur optimale parmi les premières valeurs candidates utilisées pour produire les premières images (étape 204). Le processeur 8 se base pour cela sur les premières métriques.

La première valeur candidate sélectionnée en tant que vitesse $V_{tissu1}$ au cours de l'étape 204 est celle qui a servi de donnée d'entrée pour produire une première image dont la métrique associée est indicative d'une qualité de mise au point dans la région d'intérêt considérée qui est maximale parmi toutes les premières métriques calculées. Typiquement, lorsque l'une des méthodes listées ci-dessus est utilisée pour calculer la première métrique, on recherche la première métrique de valeur maximale parmi les premières métriques calculées.

On a ainsi déterminé la vitesse $V_{tissu1}$ réelle dans le tissu biologique non osseux du corps C étudié. L'étape 104 est alors terminée.

De retour à la figure 3, le processeur 8 localise par ailleurs une première courbe de démarcation entre le tissu biologique non osseux et l'os B dans l'une des premières images construites (étape 106). Cette courbe de démarcation se situe en pratique au niveau d'une zone de l'image de forte intensité, correspondant au périoste PE.

Très préférentiellement, la localisation 106 est mise en œuvre dans la première image construite à l'aide de la première valeur candidate sélectionnée en tant que vitesse $V_{tissu1}$. La localisation est alors beaucoup plus précise du fait de la haute qualité de mise au point de cette première image parmi toutes celles qui ont été construites par le processeur 8.

La localisation 106 comprend conventionnellement les étapes suivantes :

- la première image fait l'objet d'une segmentation, de sorte à identifier un groupe de pixels de la première image montrant la frontière entre le tissu biologique non osseux et l'os B (cette segmentation comprenant par exemple la mise en œuvre d'un algorithme de Djikstra connu de l'état de la technique) ;
- ce groupe de pixels est approximé en une courbe de démarcation définie par un polynôme, par exemple une parabole.

Le processeur 8 détermine ensuite la vitesse $V_{os1}$ (étape 108) en exploitant avantageusement la première courbe de démarcation localisée dans l'étape 106, et la vitesse $V_{tissu1}$ par ailleurs déterminée au cours de l'étape 104.

En référence à la **figure 6,** la détermination 108 de la vitesse $V_{os1}$ comprend les étapes suivantes.

Pour chacune des deuxièmes valeurs candidates mémorisées dans la mémoire 10, le processeur 8 construit une deuxième image montrant du tissu osseux cortical de l'os B et l'endoste E de l'os, à partir des premiers signaux d'écho, de la vitesse $V_{tissu1}$ déterminée, et de la première courbe de démarcation, et sous l'hypothèse que la vitesse $V_{os1}$ est égale à la deuxième valeur candidate (étape 300).

La construction 300 d'une deuxième image à partir d'une deuxième valeur candidate comprend la mise en œuvre de sous-étapes similaires à celles de l'étape de construction 200 d'une première image, à quelques différences près.

Comme l'étape 200, l'étape 300 peut utiliser la méthode de migration de Kirchhoff ou la méthode dite « de mise au point totale » (« Total Focusing Method » en anglais).

Il est dans ce cas à nouveau mis en œuvre une estimation de trajectoires d'ondes passées par un point P, en exploitant à nouveau le principe de Fermât, selon lequel il est supposé qu'une onde se propage rectilignement dans un milieu homogène. Toutefois, le corps C est cette fois considéré au cours de la mise en œuvre de cette estimation de trajectoires comme un milieu hétérogène : le tissu biologique non osseux est considéré comme un premier milieu homogène, dans lequel les premières ondes ultrasonores se sont propagées à la vitesse $V_{radial\_tissu}$ préalablement déterminée. Par ailleurs, l'os B est considéré comme un deuxième milieu dans lequel ces premières ondes se sont propagées à une des deuxièmes valeurs candidates. La courbe de démarcation est ainsi une ligne d'interface entre les deux milieux, en laquelle une onde subit une réfraction.

En combinant ces hypothèses avec la courbe de démarcation préalablement localisée, il est ainsi possible d'estimer au cours de la construction 300 d'une deuxième image les trajectoires de premières ondes ultrasonores non seulement passées par un point P se trouvant dans le tissu biologique non osseux, mais encore les trajectoires de premières ondes qui sont passées par un point P se trouvant dans l'os B.

En variante, l'étape de construction 300 de la deuxième image est mise en œuvre au moyen de la méthode RTM.

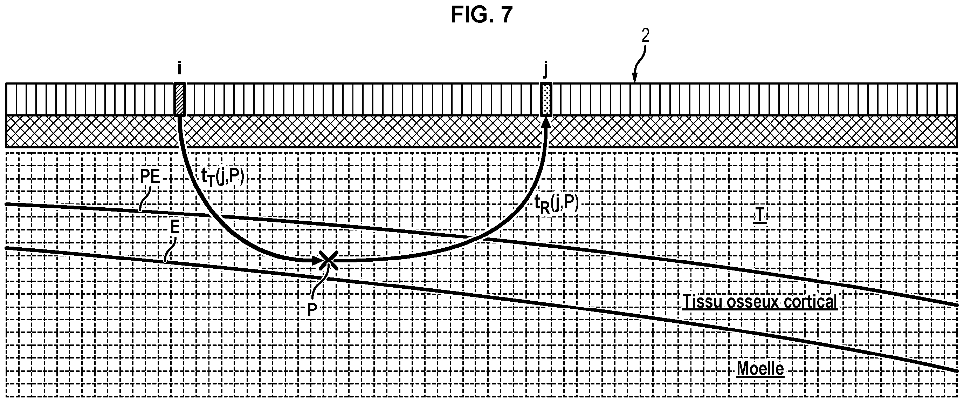

On a représenté en **figure 7** un exemple de trajectoire d'une onde ultrasonore de compression depuis l'émetteur 6 d'indice i jusqu'au récepteur 6 d'indice j, passant par un point P de l'os (plus précisément au niveau du tissu cortical de l'os), qui a été estimée au cours de la mise en œuvre de l'étape 300. Cette trajectoire estimée est continue par morceaux et comprend les quatre segments successifs suivants :

- un premier segment dans le tissu biologique non osseux T allant jusqu'à un premier point de la deuxième courbe de démarcation,
- un deuxième segment prolongeant le premier segment à partir de la deuxième courbe de démarcation jusqu'au point P, le deuxième segment étant non parallèle au premier segment du fait de la réfraction de l'onde lorsqu'elle change de milieu en le premier point de la deuxième courbe de démarcation,
- un troisième segment allant du point P vers un autre point de la deuxième courbe de démarcation, et
- un quatrième segment partant du deuxième point de la deuxième courbe de démarcation et allant vers le récepteur d'indice j, le quatrième segment étant non parallèle au troisième segment du fait de la réfraction de l'onde lorsqu'elle change à nouveau de milieu en le deuxième point de la deuxième courbe de démarcation.

Au cours de l'étape de construction 300 d'une deuxième image, le processeur 8 calcule ensuite des durées de propagation des ondes ultrasonores via les trajectoires estimées et des intensités de pixels à partir des durées de propagation, des premiers signaux d'écho et de positions des émetteurs et des récepteurs, de la même manière que celle décrite dans le cadre de la construction d'une des premières images.

Ici, la durée de propagation $t_T(i,P)$ depuis l'émetteur d'indice i jusqu'au point P couvre le premier et le deuxième segment, et la durée de propagation $t_R(j;P),i,j$ depuis le point P jusqu'au récepteur d'indice j couvre le troisième et le quatrième segment.

En définitive, la construction 300 d'une deuxième image utilise globalement les mêmes principes que la construction 200 d'une première image, mais utilise davantage de données d'entrées, notamment la courbe de démarcation, permettant ainsi aux deuxièmes images de montrer des informations exploitables au niveau de l'os B.

On a représenté en **figure 5b** un exemple de deuxième image que l'on peut obtenir par la mise en œuvre de l'étape de construction 300. Comme on peut le constater, cette deuxième image est relativement riche en information non seulement au niveau du tissu biologique non osseux T entourant l'os B, mais également à l'intérieur de l'os B, au niveau du périoste PE et au niveau de l'endoste E.

Le processeur 8 calcule ensuite, pour chaque deuxième image, au moins une deuxième métrique indicative d'une qualité de mise au point du tissu osseux cortical de l'os B et/ou de l'endoste E de l'os B dans la deuxième image (étape 302). Les troisièmes métriques sont par exemple du même type que les premières métriques.

Le processeur 8 sélectionne ensuite en tant que valeur définitive pour la vitesse $V_{os1}$ une valeur optimale parmi les deuxièmes valeurs candidates utilisées pour produire les deuxièmes images (étape 304). Le processeur 8 se base pour cela sur les deuxièmes métriques, de la même manière qu'au cours de l'étape 204.

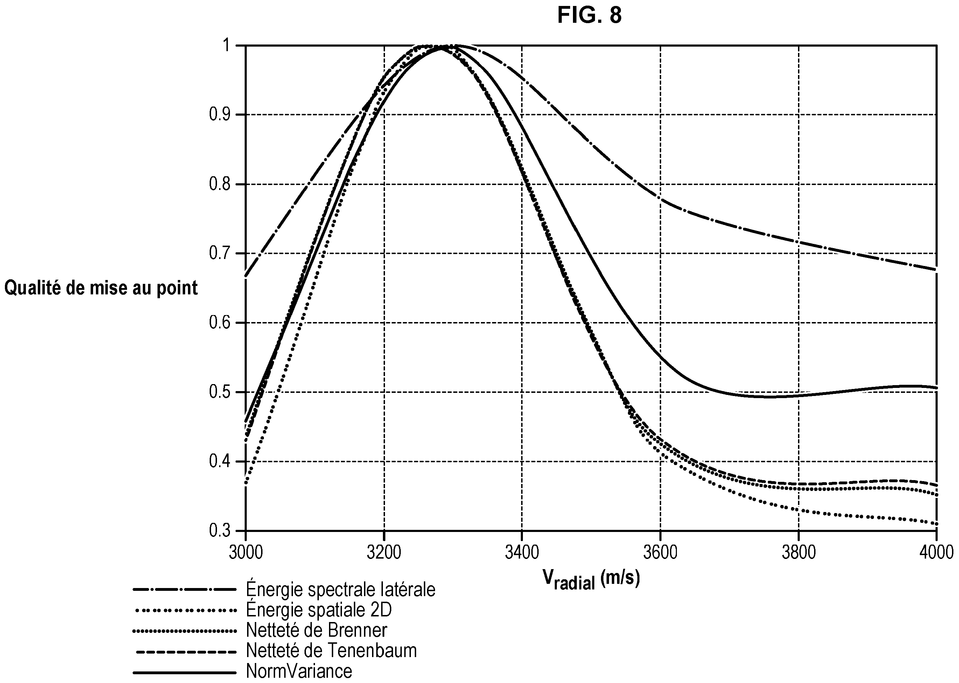

On a représenté en **figure 8** différentes courbes de deuxième métrique en fonction de la deuxième valeur candidate utilisée. On constate que, quelle que soit la méthode utilisée pour calculer la deuxième métrique, la deuxième valeur sélectionnée en tant que vitesse $V_{os1}$ est sensiblement la même (les maximas de ces courbes sont en effet très proches les uns des autres).

On a ainsi déterminé la vitesse $V_{os1}$ réelle dans l'os B du corps C étudié. L'étape 108 est alors terminée.

Cette vitesse $V_{os1}$ constitue en soi une donnée intéressante car peut servir de donnée d'entrée pour diagnostiquer une ostéoporose éventuelle de l'os B du corps C étudié.

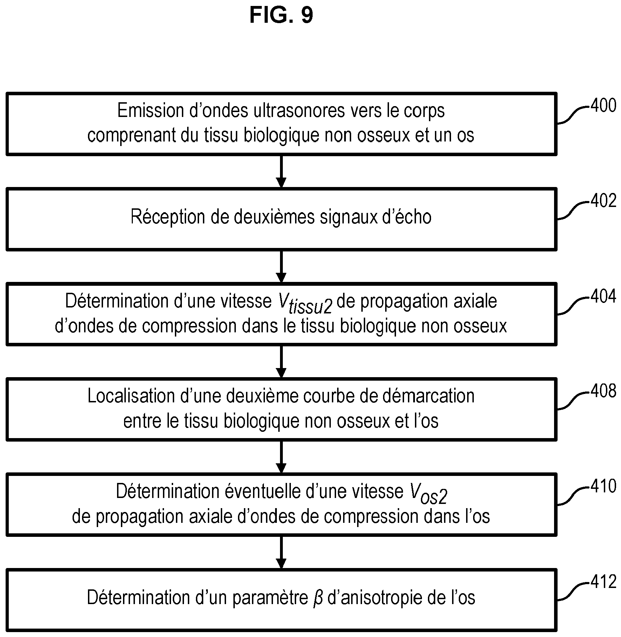

Il est toutefois avantageux de compléter cette information par d'autres vitesses afin de caractériser de manière plus complète l'os B étudié. A cette fin, le procédé mis en œuvre par le système 1 de caractérisation d'os B comprend les étapes additionnelles suivantes, en référence à la **figure 9.**

La sonde 2 est positionnée à proximité du corps C dans une deuxième position différente de la première position. Dans la deuxième position, les émetteurs-récepteurs 6 de la sonde 2 sont alignés dans une deuxième direction différente de la première direction.

La sonde 2 émet des deuxièmes ondes ultrasonores en direction du corps C dans la deuxième direction (étape 400).

Les deuxièmes ondes sont de préférence des ondes de compression axiales. Autrement dit, la deuxième direction est par exemple comprise dans un plan comprenant l'axe longitudinal de l'os B. Cette deuxième direction est de préférence sensiblement parallèle à l'axe longitudinal de l'os, c'est à dire forme un angle $\alpha$ avec l'axe longitudinal de l'os B qui est inférieur à 20 degrés.

Les deuxièmes ondes ultrasonores pénètrent dans le corps C et sont réfléchies par ce derniers à différents niveaux de profondeur dans le corps C.

Des nouveaux signaux d'écho de ces deuxièmes ondes, dits deuxièmes signaux d'écho, sont ainsi acquis par les émetteurs-récepteurs 6 de la sonde 2 (étape 402).

Les signaux d'écho sont numérisés, transmis au dispositif de traitement 4 et mémorisés dans la mémoire 10.

Eventuellement, le processeur 8 détermine ensuite sur la base de deuxièmes signaux d'écho, la vitesse $V_{os2}$ (étape 410).

L'étape 410 de détermination de la vitesse $V_{os2}$ peut être mise en œuvre au moyen d'une méthode connue de l'état de la technique se fondant sur un repérage d'une onde de tête qui se propage le long de la surface extérieure de l'os B.

Selon cette technique connue, on utilise deux deuxièmes signaux d'écho spécifiques acquis en réponse à l'émission d'ondes par deux émetteurs 6 extrémaux de la sonde 2 (typiquement, celui d'indice 0 et celui d'indice maximal). L'ensemble des émetteurs-récepteurs 6 reçoit les ondes émises sélectivement par l'une des émetteurs 6 extrémaux de la sonde 2. La distance qui sépare les deux émetteurs 6 extrémaux est connue, typiquement entre 10 et 40 mm. A cette échelle, il peut être fait l'hypothèse que la surface extérieure de l'os B est plane. Dès lors, la vitesse de propagation d'une onde de tête le long de cette surface peut être déterminée facilement étant donné que la relation entre instant d'arrivée de l'onde de tête d'une onde par l'une des deux émetteurs-récepteurs 6 utilisés et la distance séparant l'un des deux émetteurs 6 extrémaux et les récepteurs 6 est une fonction linéaire. Sous cette hypothèse de linéarité, il est très facile de déterminer :

- la vitesse V1 d'une onde de tête qui s'est propagée le long de l'os B lorsque l'émetteur-récepteur 6 d'indice 0 était utilisé en tant qu'émetteur, et que tous les émetteurs-récepteurs 6 étaient utilisés en tant que récepteurs, et
- la vitesse V2 d'une onde de tête qui s'est propagée le long de l'os B lorsque l'émetteur-récepteur 6 d'indice maximal était utilisé en tant qu'émetteur, et que tous les émetteurs-récepteurs 6 étaient utilisés en tant que récepteurs.

La vitesse $V_{os2}$ est ensuite calculée par le processeur 8 au moyen de la formule suivante :

$$V_{os2} = \frac{2V_1 V_2 \cos(\alpha)}{V_1 + V_2}$$

Par ailleurs, le processeur 8 détermine la vitesse $V_{tissu2}$ sur la base des deuxièmes signaux d'écho (étape 404). Cette étape utilise les mêmes principes fondamentaux que l'étape 104 ayant permis de déterminer la vitesse

$V_{tissu1}$, à la différence près que l'étape 404 utilise les deuxièmes ondes qui ont été émises alors que la sonde 2 occupait la deuxième position et non la première position.

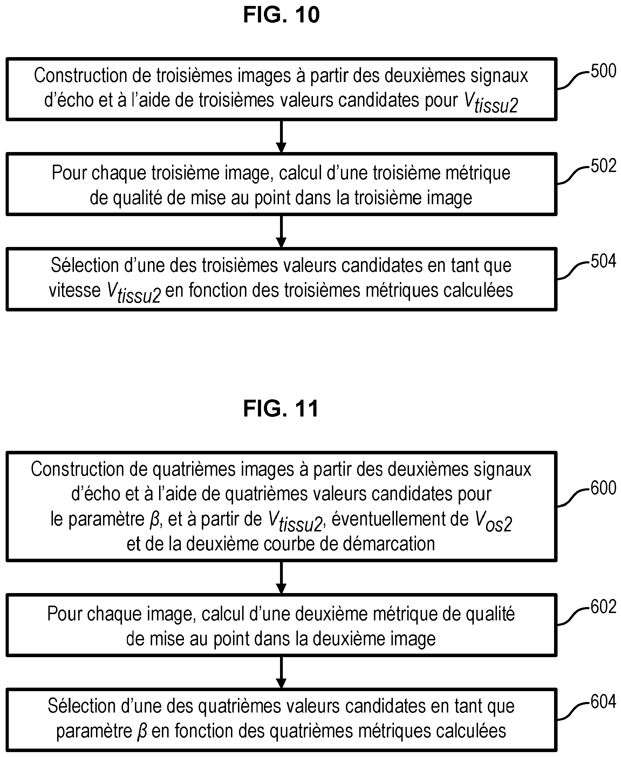

La détermination 404 de la vitesse $V_{tissu2}$ comprend plus précisément les étapes suivantes, en référence à la **figure 10** :

- pour plusieurs troisièmes valeurs candidates, construction d'une troisième image montrant le tissu biologique et le périoste PE de l'os, à partir des deuxièmes signaux d'écho et sous l'hypothèse que la vitesse $V_{tissu2}$ est égale à la deuxième valeur candidate (étape 500),
- pour chaque troisième image, calcul d'une troisième métrique indicative d'une qualité de mise au point du périoste PE dans la troisième image (étape 502),
- sélection d'une des troisièmes valeurs candidates en tant que vitesse $V_{tissu2}$, en fonction des troisièmes métriques (étape 504).

Les troisièmes images sont des vues du corps C selon une coupe longitudinale parallèle à l'axe X de l'os B.

Les troisièmes métriques sont par exemple du même type que les premières métriques et /ou des deuxièmes métriques.

De retour à la figure 9, le processeur 8 localise une deuxième courbe de démarcation entre le tissu biologique non osseux et l'os B dans l'une des troisièmes images construites (étape 408).

Très préférentiellement, la localisation 408 de la deuxième courbe est mise en œuvre dans la troisième image construite à l'aide de la troisième valeur candidate sélectionnée en tant que vitesse $V_{tissu2}$. La localisation est alors beaucoup plus précise du fait de la haute qualité de mise au point de cette troisième image parmi toutes celles qui ont été construites par le processeur 8 au cours de l'étape 500.

Le processeur 8 détermine par ailleurs le paramètre $\beta$ d'anisotropie de l'os B étudié (étape 412).

Comme indiqué précédemment, ce paramètre $\beta$ est susceptible d'être utilisé par la fonction prédéterminée V en combinaison avec la vitesse $V_{os1}$ et avec la vitesse $V_{os2}$ pour calculer une vitesse de propagation $V(\theta)$ d'une onde de compression dans l'os B suivant une direction quelconque formant un angle $\theta$ avec l'axe X.

Le paramètre $\beta$ d'anisotropie de l'os B est déterminé au cours de l'étape 412 au moyen des sous-étapes suivantes, en référence à la **figure 11.**

Pour chaque quatrième valeur candidate mémorisée dans la mémoire 10, le processeur 8 construit une quatrième image montrant du tissu osseux cortical et l'endoste E de l'os, à partir des deuxièmes signaux d'écho, de la vitesse $V_{os1}$, de la vitesse $V_{os2}$, de la fonction prédéterminée V, et sous l'hypothèse que le paramètre d'anisotropie de l'os B $\beta$ est égal à la quatrième valeur candidate (étape 600).

Cette étape 600 est similaire aux étapes 200, 300 et 500 en ce sens qu'elle peut utiliser une des méthodes de migration de Kirchhoff ou dite « de mise au point totale » (« Total Focusing Method » en anglais). Il convient de noter simplement que les trajectoires d'ondes estimées au cours de l'étape 600 se fondent dans ce cas sur l'hypothèse que la vitesse de propagation d'une onde dans l'os B observé dans une direction quelconque respecte le modèle défini par la fonction V. En variante, l'étape de construction 300 de la deuxième image est mise en œuvre au moyen de la méthode RTM.

On obtient donc à l'issue de l'étape 600 une pluralité de quatrièmes images, une pour chaque quatrième valeur candidate mémorisée initialement dans la mémoire 10.

Pour chaque quatrième image, le processeur 8 calcule au moins une quatrième métrique indicative d'une qualité de mise au point de l'endoste E et/ou du tissu biologique T entourant l'os B dans la quatrième image (étape 602). Les quatrièmes métriques sont par exemple du même type que les premières métriques.

Le processeur 8 sélectionne ensuite une des quatrièmes valeurs candidates en tant que paramètre d'anisotropie de l'os B, $\beta$, en fonction des quatrièmes métriques (étape 604).

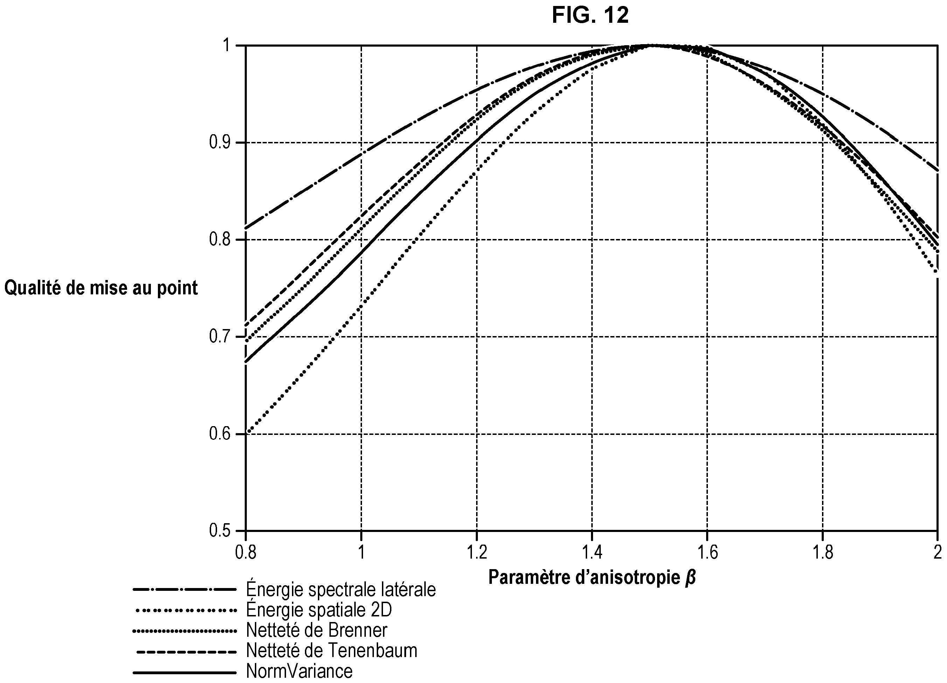

On a représenté en **figure 12** différentes courbes de quatrièmes métriques en fonction de la quatrième valeur candidate utilisée. On constate que, quelle que soit la méthode utilisée pour calculer la quatrième métrique, la quatrième valeur sélectionnée en tant que paramètre $\beta$ est sensiblement la même (les maximas de ces courbes sont en effet très proches les uns des autres), comme cela était également le cas pour la vitesse $V_{os1}$.

Il est désormais possible pour le processeur 8 de calculer n'importe quelle vitesse de propagation d'une onde de compression dans l'os B en utilisation la fonction V paramétrée avec les données $V_{os1}$, $V_{os2}$ et $\beta$ déterminées au cours du procédé.

Dans le mode de réalisation de procédé qui vient d'être décrit, les différentes données caractéristiques de l'os B que sont $V_{os1}$, $V_{os2}$, et $\beta$ ont été déterminées dans des étapes distinctes, et non de manière conjointe. En particulier, les étapes de détermination 104, 108 et 412 n'ont utilisé chacune qu'un seul ensemble de valeurs candidates (deuxièmes valeurs candidates pour l'étape 104, troisièmes valeurs candidates pour l'étape 108, quatrièmes valeurs candidates pour l'étape 412). Ceci permet en définitive de limiter le nombre de deuxièmes, troisièmes et quatrièmes images construites. Autrement dit les ressources matérielles (charge de calcul, mémoire 10) requises pour la mise en œuvre de ces étapes sont relativement modérées.

Avantageusement, le processeur 8 commande un affichage l'écran d'affichage 12 au moins une des images suivantes :

- la première image construite à l'aide de la première valeur sélectionnée au cours de l'étape 204,
- la deuxième image construite à l'aide de la deuxième valeur sélectionnée au cours de l'étape 304,
- la troisième image construite à l'aide de la première valeur sélectionnée au cours de l'étape 504,
- la deuxième image construite à l'aide de la deuxième valeur sélectionnée au cours de l'étape 604.

Ces images présentent en effet une qualité de mise au point élevée et sont par conséquent d'intérêt pour un praticien.

L'invention ne se limite pas au seul mode de réalisation qui vient d'être décrit en relation avec les figures.

L'invention est notamment applicable à l'étude d'ondes de cisaillement.

Dans ce cas, il est possible de modéliser la vitesse de propagation d'une onde de cisaillement dans l'os B au moyen de la fonction prédéterminée V de la forme suivante :

$$V(\theta) = V_{os1} \left[1 + \beta \sin^2(\theta) \cos^2(\theta)\right]$$

où

- $V_{os1}$ est une vitesse de propagation d'une onde de cisaillement dans l'os B suivant une première direction parallèle à l'axe longitudinal X,
- $\theta$ est un angle quelconque,
- $V(\theta)$ est une vitesse de propagation d'une onde de cisaillement dans l'os B suivant une direction formant l'angle $\theta$ avec la première direction.

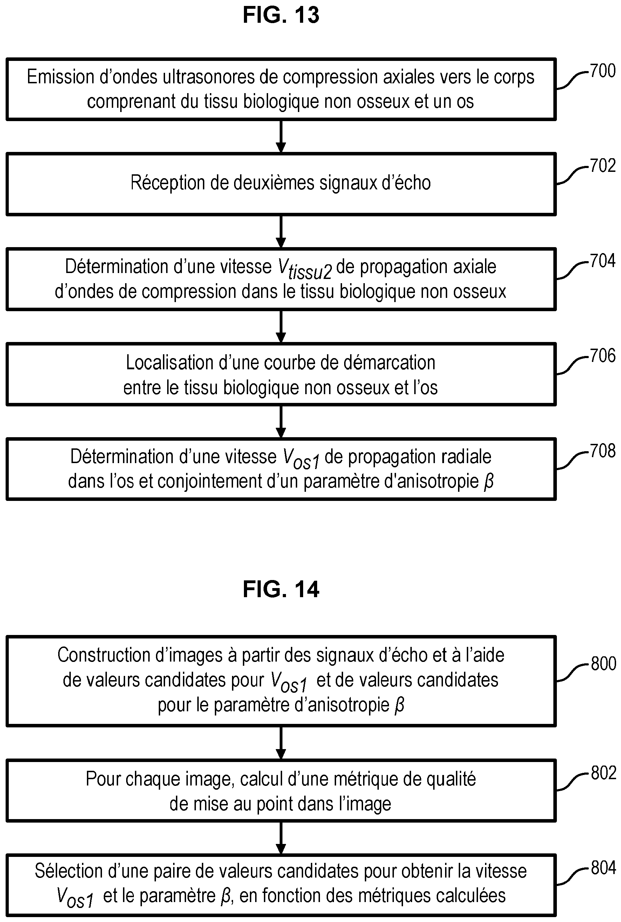

On a par exemple représenté en **figure 13** un organigramme d'étapes d'un procédé de caractérisation de l'os selon un deuxième mode de réalisation.

La sonde 2 est positionnée dans la deuxième position précédemment décrite, et des ondes ultrasonores de compression sont émises par la sonde 2 dans cette deuxième position (étape 600).

De façon connue en elle-même, les ondes de compression émises entraînent l'apparition d'ondes de cisaillement dans l'os B. Ces ondes de cisaillement sont générées lorsque les ondes de compression pénètrent dans le tissu osseux cortical de l'os B, situé entre le périoste PE et l'endoste E.

Des signaux d'écho de ces ondes de cisaillement sont ensuite reçus par la sonde 2 (étape 602).

Le processeur 8 détermine ensuite une vitesse $V_{tissu2}$ de propagation axiale d'ondes de compression dans le tissu biologique non osseux T (étape 704). Cette étape 704 est similaire à l'étape 504.

Le processeur 8 localise une courbe de démarcation entre le tissu T et l'os B (étape 706). Cette étape est identique à l'étape 408.

Le processeur 8 détermine ensuite la vitesse $V_{os1}$ (étape 708) dans l'os B, et, conjointement, le paramètre d'anisotropie $\beta$. Cette détermination conjointe comprend les sous-étapes suivantes, en référence à la **figure 14.**

On suppose qu'a été préalablement mémorisé dans la mémoire un jeu de deuxièmes valeurs candidates, pour la vitesse $V_{os1}$. Ces deuxièmes valeurs sont comprises entre 1300 et 2000 mètres par seconde pour des ondes de cisaillement dans l'os.

Par ailleurs, les quatrièmes valeurs candidates sont, dans ce mode de réalisation comprises entre -0,3 et 0.3 pour le paramètre d'anisotropie $\beta$ des ondes de cisaillement dans l'os.

Pour une paire constituée d'une quatrième valeur candidate (pour $\beta$) et d'une deuxième valeur candidate, le processeur construit une image à partir des signaux d'écho, en faisant la double hypothèse que la vitesse $V_{os1}$ est égale à la deuxième valeur candidate de la paire, et que le paramètre d'anisotropie $\beta$ est égal à la quatrième valeur de la paire (étape 800).

Le processeur 8 répète cette étape 800 pour chaque paire disponible.

On constate ici que le nombre d'images construites est bien plus grand que le nombre d'images générées au cours d'une étape du procédé selon le premier mode de réalisation, car l'on travaille sur deux dimensions au lieu d'une seule.

Pour chaque image construite, le processeur calcule une métrique de qualité de mise au point (étape 802). Cette étape est similaire aux étapes 502, 602.

Le processeur 8 sélectionne ensuite une des paires utilisées pour construire les images au cours de l'étape 800 (étape 804), sur la base des métriques calculées. Cette étape est similaire aux étapes 504, 604.

**Revendications**

1. Procédé de caractérisation d'un os (B), le procédé comprenant des étapes de :

   • émission (100, 700) de premières ondes ultrasonores vers un corps comprenant l'os (B) et du tissu biologique non osseux (T) entourant l'os (B),
   • réception (102, 702) de premiers signaux d'écho des premières ondes ultrasonores émises,
   • détermination (104, 704) d'une vitesse du son ($V_{tissu1}$) dans le tissu biologique non osseux (T) suivant une première direction, la détermination (104, 704) comprenant des sous-étapes de :

      ◦ pour plusieurs premières valeurs candidates prédéterminées, construction (200) d'une première image montrant le tissu biologique non osseux (T) et le périoste (PE) de l'os (B), à partir des premiers signaux d'écho et sous l'hypothèse que la vitesse ($V_{tissu1}$) du son dans le tissu biologique non osseux (T) suivant la première direction est égale à la première valeur candidate,
      ◦ pour chaque première image, calcul (202) d'au moins une première métrique indicative d'une qualité de mise au point du périoste et/ou du tissu biologique non osseux (T) entourant l'os dans la première image,
      ◦ sélection (204) d'une des premières valeurs candidates en tant que vitesse du son ($V_{tissu1}$) dans le tissu biologique non osseux (T) suivant la première direction, en fonction des premières métriques,

   • localisation (106, 706) d'une première courbe de démarcation entre le tissu biologique non osseux (T) et l'os dans l'une des premières images,
   • détermination (108, 708) d'une vitesse du son dans l'os ($V_{os1}$) suivant la première direction, la détermination (108, 708) comprenant des sous-étapes de :

      ◦ pour plusieurs deuxièmes valeurs candidates prédéterminées, construction (300, 800) d'une deuxième image montrant du tissu osseux cortical de l'os (B) et l'endoste (E) de l'os (B), à partir des premiers signaux d'écho, de la vitesse du son ($V_{tissu1}$) dans le tissu biologique non osseux (T) suivant la première direction déterminée, et de la première courbe de démarcation, et sous l'hypothèse que la vitesse du son dans l'os ($V_{os1}$) suivant la première direction est égale à la deuxième valeur candidate,
      ◦ pour chaque deuxième image, calcul (302, 802) d'au moins une deuxième métrique indicative d'une qualité de mise au point du tissu osseux cortical de l'os et/ou de l'endoste de l'os dans la deuxième image,
      ◦ sélection (304, 804) d'une des deuxièmes valeurs candidates en tant que vitesse du son dans l'os ($V_{os1}$) suivant la première direction, en fonction des deuxièmes métriques.

2. Procédé selon la revendication 1, dans lequel la construction (200) d'une première image à l'aide d'une première valeur candidate comprend les étapes suivantes mises en œuvre pour au moins un point du tissu biologique non osseux (T) :

   • estimation de premières trajectoires de premières ondes ultrasonores émises par des émetteurs, puis passées par le point du tissu biologique non osseux (T), puis reçues par des récepteurs, à partir des premiers signaux d'écho, sous l'hypothèse que la vitesse du son dans le tissu biologique non osseux (T) suivant la première direction est égale à la première valeur candidate,
   • calcul de durées de propagation des ondes ultrasonores via les premières trajectoires estimées,
   • calcul d'une intensité d'un pixel de la première image au point du tissu biologique non osseux (T), à partir des durées de propagation, des premiers signaux d'écho et de positions des émetteurs et des récepteurs.

3. Procédé selon l'une des revendications précédentes, dans lequel la construction (300, 800) d'une deuxième image à l'aide d'une deuxième valeur candidate comprend les étapes suivantes mises en œuvre pour au moins un point de l'os (B) :

   • estimation de deuxièmes trajectoires de premières ondes ultrasonores émises par des émetteurs, puis passées par le point de l'os, puis reçues par des récepteurs, à partir des signaux d'écho, à partir de la vitesse du son dans le tissu biologique non osseux (T) ($V_{tissu1}$) suivant la première direction déterminée, de la première courbe de démarcation, et sous l'hypothèse que la vitesse du son ($V_{os1}$) dans l'os suivant la première direction est égale à la deuxième valeur candidate,
   • calcul de durées de propagation des ondes ultrasonores via les deuxièmes trajectoires estimées,
   • calcul d'une intensité d'un pixel de la deuxième image au point de l'os (B), à partir des durées de propagation, des premiers signaux d'écho et de positions des émetteurs et des récepteurs.

**4.** Procédé selon l'une des revendications précédentes, dans lequel la localisation de la première courbe de démarcation est mise en œuvre dans la première image qui a été construite à l'aide de la première valeur sélectionnée (204) en tant que vitesse du son ($V_{tissu1}$) dans le tissu biologique non osseux (T) suivant la première direction.

**5.** Procédé selon l'une des revendications précédentes, dans lequel les premières ondes ultrasonores sont des ondes émises par des émetteurs et les signaux d'échos reçus par des récepteurs alignés le long d'un axe perpendiculaire à un axe longitudinal (X) de l'os (B), et dans lequel la première direction est une direction perpendiculaire à l'axe longitudinal (X) de l'os (B).

**6.** Procédé selon l'une des revendications précédentes, comprenant en outre un affichage de la première image construite à l'aide de la première valeur sélectionnée, et/ou de la deuxième image construite à l'aide de la deuxième valeur sélectionnée.

**7.** Procédé selon la revendication précédente, comprenant en outre des étapes de :

- émission (400) de deuxièmes ondes ultrasonores vers le corps,
- réception (402) de signaux d'écho des deuxièmes ondes ultrasonores émises, dits deuxièmes signaux d'écho,
- détermination (404) d'une vitesse du son ($V_{tissu2}$) dans le tissu biologique non osseux (T) suivant une deuxième direction différente de la première direction, comprenant des sous-étapes de :

  ◦ pour plusieurs troisièmes valeurs candidates, construction (500) d'une troisième image montrant le tissu biologique non osseux (T) et le périoste de l'os, à partir des deuxièmes signaux d'écho et sous l'hypothèse que la vitesse du son dans le tissu biologique non osseux (T) suivant la deuxième direction est égale à la deuxième valeur candidate,
  ◦ pour chaque troisième image, calcul (502) d'au moins une troisième métrique indicative d'une qualité de mise au point du périoste et/ou du tissu biologique non osseux dans la troisième image,
  ◦ sélection (504) d'une des troisièmes valeurs candidates en tant que vitesse du son ($V_{tissu2}$) dans le tissu biologique non osseux (T) suivant la deuxième direction, en fonction des troisièmes métriques,

- localisation (408) d'une deuxième courbe de démarcation entre le tissu biologique non osseux (T) et le périoste dans l'une des troisièmes images,
- détermination (410) d'une vitesse du son ($V_{os2}$) dans l'os suivant la deuxième direction, à l'aide des deuxièmes signaux d'écho,
- détermination (412) d'un paramètre d'anisotropie de l'os ($\beta$) susceptible d'être utilisé par une fonction prédéterminée (V) en combinaison avec la vitesse du son dans l'os suivant la première direction et avec la vitesse du son dans l'os suivant la deuxième direction pour calculer une vitesse du son dans l'os suivant une direction quelconque, la détermination du paramètre d'anisotropie ($\beta$) comprenant des sous-étapes de :

  ◦ pour plusieurs quatrièmes valeurs candidates prédéterminées, construction (600) d'une quatrième image montrant du tissu osseux cortical et l'endoste de l'os, à partir des deuxièmes signaux d'écho, de la vitesse de son dans l'os suivant la première direction ($V_{os1}$), de la vitesse du son ($V_{tissu2}$) dans le tissu biologique non osseux (T) suivant la deuxième direction, de la deuxième courbe de démarcation, de la fonction prédéterminée (V), et éventuellement de la vitesse de son dans l'os suivant la deuxième direction ($V_{os2}$), et sous l'hypothèse que le paramètre d'anisotropie de l'os ($\beta$) est égal à la quatrième valeur candidate,
  ◦ pour chaque quatrième image, calcul (602) d'une quatrième métrique indicative d'une qualité de mise au point de l'endoste et/ou du tissu osseux cortical de l'os dans la quatrième image,
  ◦ sélection (604) d'une des quatrièmes valeurs candidates en tant que paramètre d'anisotropie de l'os ($\beta$), en fonction des quatrièmes métriques.

**8.** Procédé selon la revendication 7, dans lequel la construction d'une quatrième image comprend les étapes suivantes mises en œuvre pour au moins un point de l'os :

- estimation de troisièmes trajectoires de deuxièmes ondes ultrasonores émises par des émetteurs, puis passées par le point de l'os, puis reçues par des récepteurs, à partir des deuxièmes signaux d'écho, de la vitesse du son ($V_{tissu2}$) dans le tissu biologique non osseux (T) suivant la deuxième direction, de la vitesse de son dans l'os ($V_{os1}$) suivant la première direction déterminée, de la deuxième courbe de démarcation, de la fonction prédéterminée (V), et éventuellement de la vitesse du son dans l'os ($V_{os2}$) suivant la deuxième direction déterminée, et sous l'hypothèse que le paramètre d'anisotropie de l'os ($\beta$) est égale à la quatrième valeur candidate,

• calcul de durées de propagation des ondes ultrasonores via les troisièmes trajectoires estimées,
• calcul d'une intensité d'un pixel de la quatrième image au point de l'os, à partir des durées de propagation et des deuxièmes signaux d'écho et de positions des émetteurs et des récepteurs.

9. Procédé selon l'une des revendication 7 à 8, dans lequel la localisation (408) de la deuxième courbe de démarcation est mise en œuvre dans la troisième image ayant été construite à l'aide de la troisième valeur sélectionnée en tant que vitesse du son dans le tissu biologique non osseux (T) suivant la deuxième direction.

10. Procédé selon l'une des revendication 7 à 9, comprenant en outre un affichage de la troisième image construite à l'aide de la troisième valeur sélectionnée, et/ou de la quatrième image construite à l'aide de la quatrième valeur sélectionnée et de la vitesse du son dans l'os suivant la deuxième direction ($V_{os2}$).

11. Procédé selon l'une des revendications 7 à 10, dans lequel les deuxièmes ondes ultrasonores sont des ondes émises par des émetteurs et les deuxièmes signaux d'écho sont reçus par des récepteurs alignés le long d'un axe compris dans un plan comprenant par ailleurs un axe longitudinal (X) de l'os (B), et dans lequel la deuxième direction est de préférence une direction parallèle à l'axe longitudinal (X) de l'os (B).

12. Procédé selon l'une des revendications précédentes, dans lequel la ou chaque vitesse du son dans l'os ($V_{os1}$, $V_{os2}$) déterminée est une vitesse de propagation d'ondes de compression ou une vitesse de propagation d'ondes de cisaillement.

13. Procédé selon l'une des revendications 7 à 12, dans lequel la ou chaque vitesse du son dans l'os ($V_{os1}$, $V_{os2}$) déterminée est une vitesse de propagation d'ondes de compression, et dans lequel la fonction prédéterminée est une fonction V de la forme :

$$V(\theta) = V_{os2} - (V_{os2} - V_{os1})[\beta \sin^2(\theta) \cos^2(\theta) + \cos^4(\theta)]$$

dans lequel

• $V_{os1}$ est la vitesse de propagation d'ondes de compression dans l'os suivant la première direction déterminée,
• $V_{os2}$ est la vitesse de propagation d'ondes de compression dans l'os suivant la deuxième direction déterminée,
• $\beta$ est le paramètre d'anisotropie de l'os,
• $\theta$ est un angle,
• $V(\theta)$ est une vitesse de propagation d'ondes de compression dans l'os suivant une direction formant l'angle $\theta$ avec la première direction.

14. Procédé selon l'une des revendications précédentes, dans lequel la vitesse de son dans l'os suivant la première direction déterminée est une vitesse de propagation d'ondes de cisaillement, et dans lequel la fonction prédéterminée est une fonction V de la forme :

$$V(\theta) = V_{os1} [1 + \beta \sin^2(\theta) \cos^2(\theta)]$$

dans lequel

• $V_{os1}$ est la vitesse de propagation d'ondes de cisaillement dans l'os suivant la première direction déterminée,
• $\beta$ est le paramètre d'anisotropie de l'os,
• $\theta$ est un angle,
• $V(\theta)$ est une vitesse de propagation d'ondes de cisaillement dans l'os suivant une direction formant l'angle $\theta$ avec la première direction.

15. Procédé selon l'une des revendications 1 à 7, dans lequel il est déterminé (708), conjointement à la vitesse ($V_{os1}$) du son dans l'os suivant la première direction, un paramètre d'anisotropie de l'os ($\beta$) susceptible d'être utilisé par une fonction prédéterminée en combinaison avec la vitesse du son dans l'os suivant la première direction déterminée pour calculer une vitesse du son dans l'os suivant une direction quelconque, ladite détermination conjointe comprenant des sous-étapes de :

• pour plusieurs paires de deuxièmes et quatrièmes valeurs candidates prédéterminées, construction (800) d'une deuxième image montrant du tissu osseux cortical de l'os et l'endoste de l'os, à partir des premiers signaux d'écho, de la vitesse du son dans le tissu biologique non osseux suivant la première direction déterminée, et de la première courbe de démarcation, sous l'hypothèse que la vitesse du son dans l'os suivant la première direction est égale à la deuxième valeur candidate, et sous l'hypothèse que le paramètre d'anisotropie de l'os ($\beta$) est égal à la quatrième valeur candidate,
• pour chaque deuxième image, calcul (802) d'au moins une deuxième métrique indicative d'une qualité de mise au point du tissu osseux cortical de l'os et/ou de l'endoste de l'os dans la deuxième image,
• sélection (804) conjointe d'une deuxième valeur candidate en tant que vitesse du son dans l'os suivant la première direction, et d'une quatrième valeur candidate en tant que paramètre d'anisotropie de l'os ($\beta$), la sélection étant mise en œuvre en fonction des deuxièmes métriques.

**Patentansprüche**

1.  Charakterisierungsverfahren eines Knochens (B), wobei das Verfahren die Schritte umfasst:

    • Senden (100, 700) von ersten Ultraschallwellen zu einem Körper, umfassend den Knochen (B) und nicht knochenhaltiges biologisches Gewebe (T), das den Knochen (B) umgibt,
    • Empfangen (102, 702) von ersten Echosignalen der ersten gesendeten Ultraschallwellen,
    • Bestimmen (104, 704) einer Geschwindigkeit des Schalls ($V_{tissu1}$) in dem nicht knochenhaltigen biologischen Gewebe (T) gemäß einer ersten Richtung, wobei das Bestimmen (104, 704) die Teilschritte umfasst:

        ◦ für mehrere erste vorbestimmte Kandidaten Aufbauen (200) eines ersten Bildes, das das nicht knochenhaltige biologische Gewebe (T) und die Knochenhaut (PE) des Knochens (B) ausgehend von den ersten Echosignalen und in der Annahme zeigt, dass die Geschwindigkeit ($V_{tissue1}$) des Schalls in dem nicht knochenhaltigen biologischen Gewebe (T) gemäß der ersten Richtung gleich dem ersten Kandidatenwert ist,
        ◦ für jedes erste Bild Berechnen (202) wenigstens einer ersten Metrik, die eine Einstellungsqualität der Knochenhaut und/oder des nicht knochenhaltigen biologischen Gewebes (T) anzeigt, das den Knochen in dem ersten Bild umgibt,
        ◦ Auswählen (204) eines der ersten Kandidatenwerte als Geschwindigkeit des Schalls ($V_{tissue1}$) in dem nicht knochenhaltigen biologischen Gewebe (T) gemäß der ersten Richtung in Abhängigkeit von den ersten Metriken,

    • Lokalisieren (106, 706) einer ersten Demarkationskurve zwischen dem nicht knochenhaltigen biologischen Gewebe (T) und dem Knochen in einem der ersten Bilder,
    • Bestimmen (108, 708) einer Geschwindigkeit des Schalls in dem Knochen ($V_{os1}$) gemäß der ersten Richtung, wobei das Bestimmen (108, 708) die Teilschritte umfasst:

        ◦ für mehrere zweite vorbestimmte Kandidatenwerte Aufbauen (300, 800) eines zweiten Bildes, das kortikales Knochengewebe des Knochens (B) und der Knochenhaut (E) des Knochens (B), ausgehend von den ersten Echosignalen, der Geschwindigkeit des Schalls ($V_{tissu1}$) in dem nicht knochenhaltigen biologischen Gewebe (T) gemäß der ersten bestimmten Richtung und der ersten Demarkationskurve und in der Annahme zeigt, dass die Geschwindigkeit des Schalls in dem Knochen ($V_{os1}$) gemäß der ersten Richtung gleich dem zweiten Kandidatenwert ist,
        ◦ für jedes zweite Bild Berechnen (302, 802) wenigstens einer zweiten Metrik, die eine Einstellungsqualität des kortikalen Knochengewebes des Knochens und/ oder des Endosteums des Knochens in dem zweiten Bild anzeigt,
        ◦ Auswählen (304, 804) einer der zwei Kandidatenwerte als Geschwindigkeit des Schalls in dem Knochen ($V_{os1}$) gemäß der ersten Richtung in Abhängigkeit von den zweiten Metriken.

2.  Verfahren gemäß Anspruch 1, bei dem das Aufbauen (200) eines ersten Bildes mithilfe eines ersten Kandidatenwertes die folgenden Schritte umfasst, die für wenigstens einen Punkt des nicht knochenhaltigen biologischen Gewebes (T) umgesetzt werden:

    • Schätzen von ersten Bahnen erster Ultraschallwellen, die von Sendern gesendet werden, dann von dem Punkt des nicht knochenhaltigen biologischen Gewebes (T) durchlaufen werden, dann von Empfängern ausgehend von ersten Echosignalen empfangen werden, in der Annahme, dass die Geschwindigkeit des Schalls in dem nicht knochenhaltigen biologischen Gewebe (T) gemäß der ersten Richtung gleich dem ersten Kandidatenwert ist,
• Berechnen von Verbreitungsdauern der Ultraschallwellen über die ersten geschätzten Bahnen,
• Berechnen einer Intensität eines Pixels des ersten Bildes am Punkt des nicht knochenhaltigen biologischen Gewebes (T) ausgehend von den Verbreitungsdauern, der ersten Echosignale und Sender- und Empfängerpositionen.

3. Verfahren gemäß irgendeinem der voranstehenden Ansprüche, bei dem das Aufbauen (300, 800) eines zweiten Bildes mithilfe eines zweiten Kandidatenwertes die folgenden Schritte umfasst, die für wenigstens einen Punkt des Knochens (B) umgesetzt werden:

   • Schätzen von zweiten Bahnen von ersten Ultraschallwellen, die von Sendern versendet werden, dann ausgehend von Echosignalen von dem Punkt des Knochens durchlaufen werden, dann von Empfängern empfangen werden, ausgehend von der Geschwindigkeit des Schalls in dem nicht knochenhaltigen biologischen Gewebe (T) ($V_{tissu1}$) gemäß der ersten bestimmten Richtung der ersten Demarkationskurve und in der Annahme, dass die Geschwindigkeit des Schalls ($V_{os1}$) in dem Knochen gemäß der ersten Richtung gleich dem zweiten Kandidatenwert ist,
   • Berechnen von Verbreitungsdauern der Ultraschallwellen über die zweiten geschätzten Bahnen,
   • Berechnen einer Intensität eines Pixels des zweiten Bildes am Punkt des Knochens (B) ausgehend von den Verbreitungsdauern, den ersten Echosignalen und Sender- und Empfängerpositionen.

4. Verfahren gemäß irgendeinem der voranstehenden Ansprüche, bei dem die Lokalisation der ersten Demarkationskurve in dem ersten Bild umgesetzt wird, das mithilfe des ersten ausgewählten Wertes (204) als Geschwindigkeit des Schalls ($V_{tissu1}$) in dem nicht knochenhaltigen biologischen Gewebe (T) gemäß der ersten Richtung aufgebaut wird.

5. Verfahren gemäß irgendeinem der voranstehenden Ansprüche, bei dem die ersten Ultraschallwellen Wellen sind, die von Sendern gesendet werden und die Echosignale von Empfängern empfangen werden, die entlang einer zu einer Längsachse (X) des Knochens (B) lotrechten Achse angeordnet sind, und bei dem die erste Richtung eine zur Längsachse (X) des Knochens (B) lotrechte Richtung ist.

6. Verfahren gemäß einem der voranstehenden Ansprüche, umfassend darüber hinaus eine Anzeige des ersten Bildes, das mithilfe des ersten ausgewählten Wertes aufgebaut wird und/oder des zweiten Bildes, das mithilfe des zweiten ausgewählten Wertes aufgebaut wird.

7. Verfahren gemäß dem voranstehenden Anspruch, umfassend darüber hinaus die Schritte:

   • Senden (400) von zweiten Ultraschallwellen zu dem Körper,
   • Empfangen (402) von Echosignalen der zweiten gesendeten Ultraschallwellen, bezeichnet als zweite Echosignale,
   • Bestimmen (404) einer Geschwindigkeit des Schalls ($V_{tissu2}$) in dem nicht knochenhaltigen biologischen Gewebe (T) gemäß einer von der ersten Richtung unterschiedlichen zweiten Richtung, umfassend die Teilschritte:

      ◦ für mehrere dritte Kandidatenwerte Aufbauen (500) eines dritten Bildes, das das nicht knochenhaltige biologische Gewebe (T) und die Knochenhaut des Knochens zeigt, ausgehend von zweiten Echosignalen und in der Annahme, dass die Geschwindigkeit des Schalls in dem nicht knochenhaltigen biologischen Gewebe (T) gemäß der zweiten Richtung gleich dem zweiten Kandidatenwert ist,
      ◦ für jedes dritte Bild Berechnen (502) wenigstens einer dritten Metrik, die eine Einstellungsqualität der Knochenhaut und/oder des nicht knochenhaltigen biologischen Gewebes in dem dritten Bild anzeigt,
      ◦ Auswählen (504) eines der dritten Kandidatenwerte als Geschwindigkeit des Schalls ($V_{tissu2}$) in dem nicht knochenhaltigen biologischen Gewebe (T) gemäß der zweiten Richtung in Abhängigkeit von den dritten Metriken,

   • Lokalisieren (408) einer zweiten Demarkationskurve zwischen dem nicht knochenhaltigen biologischen Gewebe (T) und der Knochenhaut in einem der dritten Bilder,
   • Bestimmen (410) einer Geschwindigkeit des Schalls ($V_{os2}$) in dem Knochen gemäß der zweiten Richtung mithilfe von zweiten Echosignalen,
   • Bestimmen (412) eines Anisotropieparameters des Knochens (ß), der von einer vorbestimmten Funktion (V) in Kombination mit der Geschwindigkeit des Schalls in dem Knochen gemäß der ersten Richtung und mit der Geschwindigkeit des Schalls gemäß der zweiten Richtung verwendet werden kann, um eine Geschwindigkeit des Schalls in dem Knochen gemäß einer beliebigen Richtung zu berechnen, wobei die Bestimmung des Anisotropieparameters ($\beta$) Teilschritte umfasst:

◦ für mehrere vierte vorbestimmte Kandidatenwerte Aufbauen (600) eines vierten Bildes, das kortikales Knochengewebe und das Endosteum des Knochens zeigt, ausgehend von zweiten Echosignalen, der Geschwindigkeit des Schalls in dem Knochen gemäß der ersten Richtung ($V_{os1}$), der Geschwindigkeit des Schalls ($V_{tissu2}$) in dem nicht knochenhaltigen biologischen Gewebe (T) gemäß der zweiten Richtung, der zweiten Demarkationskurve, der vorbestimmten Funktion (V) und eventuell der Geschwindigkeit des Schalls in dem Knochen gemäß der zweiten Richtung ($V_{os2}$) und in der Annahme, dass der Anisotropieparameter des Knochens ($\beta$) gleich dem vierten Kandidatenwert ist,
◦ für jedes vierte Bild Berechnen (602) einer vierten Metrik, die eine Einstellungsqualität des Endosteums und/oder des kortikalen Knochengewebes des Knochens in dem vierten Bild anzeigt,
◦ Auswählen (604) eines der vierten Kandidatenwerte als Anisotropieparameter des Knochens ($\beta$) in Abhängigkeit von den vierten Metriken.

8. Verfahren gemäß Anspruch 7, bei dem das Aufbauen eines vierten Bildes die folgenden Schritte umfasst, die für wenigstens einen Punkt des Knochens umgesetzt werden:

• Schätzen von dritten Bahnen von zweiten Ultraschallwellen, die von Sendern versendet werden, dann von dem Punkt des Knochens durchlaufen werden, dann von Empfängern, ausgehend von zweiten Echosignalen, der Geschwindigkeit des Schalls ($V_{tiss2}$) in dem nicht knochenhaltigen biologischen Gewebe (T) gemäß der zweiten Richtung, der Geschwindigkeit des Schalls in dem Knochen ($V_{os1}$) gemäß der ersten bestimmten Richtung, der zweiten Demarkationskurve, der vorbestimmten Funktion (V) und eventuell der Geschwindigkeit des Schalls in dem Knochen ($V_{os2}$) gemäß der zweiten bestimmten Richtung empfangen werden und in der Annahme, dass der Anisotropieparameter des Knochens ($\beta$) gleich dem vierten Kandidatenwert ist,
• Berechnen von Verbreitungsdauern der Ultraschallwellen über die dritten geschätzten Bahnen,
• Berechnen einer Intensität eines Pixels des vierten Bildes am Punkt des Knochens ausgehend von den Verbreitungsdauern und den zweiten Echo- und Positionssignalen der Sender und Empfänger.

9. Verfahren gemäß einem der Ansprüche 7 bis 8, bei dem das Lokalisieren (408) der zweiten Demarkationskurve in dem dritten Bild umgesetzt wird, das mithilfe des dritten ausgewählten Wertes als Geschwindigkeit des Schalls in dem nicht knochenhaltigen biologischen Gewebe (T) gemäß der zweiten Richtung aufgebaut wurde.

10. Verfahren gemäß einem der Ansprüche 7 bis 9, umfassend darüber hinaus eine Anzeige des dritten Bildes, das mithilfe des dritten ausgewählten Wertes aufgebaut wurde und/oder des vierten Bildes, das mithilfe des vierten ausgewählten Wertes aufgebaut wurde, und der Geschwindigkeit des Schalls in dem Knochen gemäß der zweiten Richtung (Vos2).

11. Verfahren gemäß einem der Ansprüche 7 bis 10, bei dem die zweiten Ultraschallwellen Wellen sind, die von Sendern gesendet werden, und zweite Echosignale sind, die von Empfängern empfangen werden, die entlang einer Achse angeordnet sind, die in einer Ebene umfasst ist, umfassend im Übrigen eine Längsachse (X) des Knochens (B) und bei dem die zweite Richtung bevorzugt eine zur Längsachse (X) des Knochens (B) parallele Richtung ist.

12. Verfahren gemäß einem der voranstehenden Ansprüche, bei dem die oder jede bestimmte Geschwindigkeit des Schalls in dem Knochen ($V_{os1}$, $V_{os2}$) eine Verbreitungsgeschwindigkeit von Kompressionswellen oder eine Verbreitungsgeschwindigkeit von Abscherwellen ist.

13. Verfahren gemäß einem der Ansprüche 7 bis 12, bei dem die oder jede bestimmte Geschwindigkeit des Schalls in dem Knochen ($V_{os1}$, $V_{os2}$) eine Verbreitungsgeschwindigkeit von Kompressionswellen ist und bei dem die vorbestimmte Funktion eine Funktion V mit der Formel ist:

$$V(\theta) = V_{os2} - (V_{os2} - V_{os1}) [\beta \sin^2(\theta) \cos^2(\theta) + \cos^4(\theta)]$$

in der

• V$_{os1}$ die Verbreitungsgeschwindigkeit von Kompressionswellen in dem Knochen gemäß der ersten bestimmten Richtung ist,
• V$_{os2}$ die Verbreitungsgeschwindigkeit von Kompressionswellen in dem Knochen gemäß der zweiten bestimmten Richtung ist,
• β der Anisotropieparameter des Knochens ist,
• θ ein Winkel ist,
• V (θ) eine Verbreitungsgeschwindigkeit von Kompressionswellen in dem Knochen gemäß einer Richtung ist, die mit der ersten Richtung den Winkel θ bildet.

14. Verfahren gemäß einem der voranstehenden Ansprüche, bei dem die Geschwindigkeit des Schalls in dem Knochen gemäß der ersten bestimmten Richtung eine Verbreitungsgeschwindigkeit von Abscherwellen ist und bei dem die vorbestimmte Funktion eine Funktion V der Form ist:

$$V(\theta) = V_{os1} [1 + \beta \sin^2 (\theta) \cos^2 (\theta)]$$

in der

• V$_{os1}$ die Verbreitungsgeschwindigkeit von Abscherwellen in dem Knochen gemäß der ersten bestimmten Richtung ist,
• β der Anisotropieparameter des Knochens ist,
• θ ein Winkel ist,
• V (θ) eine Verbreitungsgeschwindigkeit von Abscherwellen in dem Knochen gemäß einer Richtung ist, die mit der ersten Richtung den Winkel θ bildet.

15. Verfahren gemäß einem der Ansprüche 1 bis 7, bei dem in Verbindung mit der Geschwindigkeit (V$_{os1}$) des Schalls in dem Knochen gemäß der ersten Richtung ein Anisotropieparameter des Knochens (β) bestimmt wird (708), der geeignet ist, von einer vorbestimmten Funktion in Kombination mit der Geschwindigkeit des Schalls in dem Knochen gemäß der ersten bestimmten Richtung verwendet zu werden, um eine Geschwindigkeit des Schalls in dem Knochen gemäß einer beliebigen Richtung zu berechnen, wobei die genannte gemeinsame Bestimmung Teilschritte umfasst:

• für mehrere Paare von zweiten und vierten vorbestimmten Kandidatenwerten Aufbauen (800) eines zweiten Bildes, das kortikales Knochengewebe des Knochens und das Endosteum des Knochens zeigt, ausgehend von ersten Echosignalen, der Geschwindigkeit des Schalls in dem nicht knochenhaltigen biologischen Gewebe gemäß der ersten vorbestimmten Richtung und der ersten Demarkationskurve in der Annahme, dass die Geschwindigkeit des Schalls in dem Knochen gemäß der ersten Richtung gleich dem zweiten Kandidatenwert ist und in der Annahme, dass der Anisotropieparameter des Knochens (β) gleich dem vierten Kandidatenwert ist,
• für jedes zweite Bild Berechnen (802) wenigstens einer zweiten Metrik, die eine Einstellungsqualität des kortikalen Knochengewebes des Knochens und/oder des Endosteums des Knochens in dem zweiten Bild anzeigt,
• gemeinsames Auswählen (804) eines zweiten Kandidatenwertes als Geschwindigkeit des Schalls in dem Knochen gemäß der ersten Richtung und eines vierten Kandidatenwertes als Anisotropieparameter des Knochens (β), wobei die Auswahl in Abhängigkeit von den zweiten Metriken umgesetzt wird.

**Claims**

1. Method for characterising bone (B), the method comprising the steps of:

• transmitting (100, 700) first ultrasonic waves to a body comprising bone (B) and non-bone biological tissue (T) surrounding bone (B),
• receiving (102, 702) first echo signals of the first ultrasonic waves transmitted,
• determining (104, 704) a speed of sound ($V_{tissu1}$) in non-bone biological tissue (T) in a first direction, the determining (104, 704) comprising the sub-steps of:

  ○ for several first predetermined candidate values, constructing (200) a first image showing non-bone biological tissue (T) and the periosteum (PE) of the bone (B), from the first echo signals and under the hypothesis that the speed ($V_{tissu1}$) of sound in non-bone biological tissue (T) in the first direction is equal to the first candidate value,

○ for each first image, calculating (202) at least one first metric indicative of a focus quality of the periosteum and/or of the non-bone biological tissue (T) surrounding bone in the first image,

○ selecting (204) of one of the first candidate values as the speed of sound ($V_{tissu1}$) in non-bone biological tissue (T) in the first direction, according to the first metrics,

• locating (106, 706) a first demarcation curve between the non-bone biological tissue (T) and the bone in one of the first images,

• determining (108, 708) a speed of sound in bone ($V_{os1}$) in the first direction, the determining (108, 708) comprising the sub-steps of:

○ for several second predetermined candidate values, constructing (300, 800) a second image showing cortical bone tissue of the bone (B) and the endosteum (E) of the bone (B), from the first echo signals, the speed of sound ($V_{tissu1}$) in non-bone biological tissue (T) in the first determined direction, and the first demarcation curve, and under the hypothesis that the speed of sound in bone ($V_{os1}$) in the first direction is equal to the second candidate value,

○ for each second image, calculating (302, 802) at least one second metric indicative of a focus quality of the cortical bone tissue of the bone and/or of the endosteum of the bone in the second image,

○ selecting (304, 804) one of the second candidate values as the speed of sound in bone ($V_{os1}$) in the first direction, according to the second metrics.

2.  Method according to claim 1, wherein the constructing (200) of a first image using a first candidate value comprises the following steps implemented for at least one point of the non-bone biological tissue (T):

• estimating first trajectories of first ultrasonic waves transmitted by transmitters, then passed through the point of the non-bone biological tissue (T), then received by receptors, from the first echo signals, under the hypothesis that the speed of sound in non-bone biological tissue (T) in the first direction is equal to the first candidate value,

• calculating propagation durations of ultrasonic waves via the first estimated trajectories,

• calculating an intensity of a pixel of the first image at the point of the non-bone biological tissue (T), from propagation durations, first echo signals and positions of the transmitters and the receptors.

3.  Method according to one of the preceding claims, wherein the constructing (300, 800) of a second image using a second candidate value comprises the following steps implemented for at least one point of the bone (B):

• estimating second trajectories of first ultrasonic waves transmitted by transmitters, then passed through the point of the bone, then received by receptors, from echo signals, from the speed of sound in the non-bone biological tissue (T) ($V_{tissu1}$) in the first determined direction, of the first demarcation curve, and under the hypothesis that the speed of sound ($V_{os1}$) in bone in the first direction is equal to the second candidate value,

• calculating propagation durations of the ultrasonic waves via the second estimated trajectories,

• calculating an intensity of a pixel of the second image at the point of the bone (B), from propagation durations, of the first echo signals and of positions of the transmitters and the receptors.

4.  Method according to one of the preceding claims, wherein the locating of the first demarcation curve is implemented in the first image that was constructed using the first value selected (204) as the speed of sound ($V_{tissu1}$) in non-bone biological tissue (T) in the first direction.

5.  Method according to one of the preceding claims, wherein the first ultrasonic waves are waves transmitted by transmitters and the echo signals received by receptors aligned along an axis perpendicular to a longitudinal axis (X) of the bone (B), and wherein the first direction is a direction perpendicular to the longitudinal axis (X) of the bone (B) .

6.  Method according to one of the preceding claims, further comprising displaying the first image constructed using the first value selected, and/or the second image constructed using the second value selected.

7.  Method according to the preceding claim, further comprising steps of:

• transmitting (400) second ultrasonic waves to the body,

• receiving (402) echo signals of the second ultrasonic waves transmitted, referred to as second echo signals,

• determining (404) a speed of sound ($V_{tissu2}$) in non-bone biological tissue (T) in a second direction different from the first direction, comprising the sub-steps of:

◦ for several third candidate values, constructing (500) of a third image showing the non-bone biological tissue (T) and the periosteum of the bone, from the second echo signals and under the hypothesis that the speed of sound in non-bone biological tissue (T) in the second direction is equal to the second candidate value,
◦ for each third image, calculating (502) at least one third metric indicative of a focus quality of the periosteum and/or of the non-bone biological tissue in the third image,
◦ selecting (504) one of the three candidate values as the speed of sound ($V_{tissu2}$) in non-bone biological tissue (T) in the second direction, according to the third metrics,

• locating (408) a second demarcation curve between the non-bone biological tissue (T) and the periosteum in one of the third images,
• determining (410) a speed of sound ($V_{os2}$) in bone in the second direction, using the second echo signals,
• determining (412) an anisotropy parameter of the bone (β) able to be used by a predetermined function (V) in combination with the speed of sound in bone in the first direction and with the speed of sound in bone in the second direction to calculate a speed of sound in bone in any direction, the determining of the anisotropy parameter (β) comprising the sub-steps of:

◦ for several fourth predetermined candidate values, constructing (600) a fourth image showing cortical bone tissue and the endosteum of the bone, from the second echo signals, the speed of sound in bone in the first direction (Vos1), the speed of sound (Vtissu2) in non-bone biological tissue (T) in the second direction, the second demarcation curve, the predetermined function (V), and optionally the speed of sound in bone in the second direction (Vos2), and under the hypothesis that the anisotropy parameter of the bone (β) is equal to the fourth candidate value,
◦ for each fourth image, calculating (602) a fourth metric indicative of a focus quality of the endosteum and/or of the cortical bone tissue of the bone in the fourth image,
◦ selecting (604) one of the fourth candidate values as an anisotropy parameter of the bone (β), according to the fourth metrics.

**8.** Method according to claim 7, wherein the constructing of a fourth image comprises the following steps implemented for at least one point of the bone:

• estimating third trajectories of second ultrasonic waves transmitted by transmitters, then passed through the point of the bone, then received by receptors, from second echo signals, the speed of sound ($V_{tissu2}$) in non-bone biological tissue (T) in the second direction, the speed of sound in bone ($V_{os1}$) in the first determined direction, the second demarcation curve, the predetermined function (V), and optionally the speed of sound in bone ($V_{os2}$) in the second determined direction, and under the hypothesis that the anisotropy parameter of the bone (β) is equal to the fourth candidate value,
• calculating propagation durations of ultrasonic waves via the third estimated trajectories,
• calculating an intensity of a pixel of the fourth image at the point of the bone, from propagation durations and second echo signals and positions of the transmitters and the receptors.

**9.** Method according to one of claims 7 to 8, wherein the locating (408) of the second demarcation curve is implemented in the third image having been constructed using the third value selected as the speed of sound in non-bone biological tissue (T) in the second direction.

**10.** Method according to one of claims 7 to 9, further comprising a displaying of the third image constructed using the third value selected, and/or of the fourth image constructed using the fourth value selected and of the speed of sound in bone in the second direction ($V_{os2}$) .

**11.** Method according to one of claims 7 to 10, wherein the second ultrasonic waves are waves transmitted by transmitters and the second echo signals are received by receptors aligned along an axis comprised in a plane comprising moreover a longitudinal axis (X) of the bone (B), and wherein the second direction is preferably a direction parallel to the longitudinal axis (X) of the bone (B).

**12.** Method according to one of the preceding claims, wherein the or each speed of sound in bone ($V_{os1}$) , ($V_{os2}$)

determined is a compression wave propagation speed or a shearing wave propagation speed.

**13.** Method according to one of claims 7 to 12, wherein the or each speed of sound in bone ($V_{os1}$), ($V_{os2}$) determined is a compression wave propagation speed, and wherein the predetermined function is a function V of the form:

$$V(\theta) = V_{os2} - (V_{os2} - V_{os1})[\beta \sin^2(\theta) + \cos^4(\theta)]$$

wherein

- $V_{os1}$ is the compression wave propagation speed in bone in the first determined direction,
- $V_{os2}$ the compression wave propagation speed in bone in the second determined direction,
- $\beta$ is the anisotropy parameter of the bone,
- $\theta$ is an angle,
- $V(\theta)$ is a compression wave propagation speed in bone in a direction forming the angle $\theta$ with the first direction.

**14.** Method according to one of the preceding claims, wherein the speed of sound in bone in the first determined direction is a shearing wave propagation speed, and wherein the predetermined function is a function V of the form:

$$V(\theta) = V_{os1}[1 + \beta \sin^2(\theta) \cos^4(\theta)]$$

wherein

- $V_{os1}$ is the shearing wave propagation speed in bone in the first determined direction,
- $\beta$ is the anisotropy parameter of the bone,
- $\theta$ is an angle,
- $V(\theta)$ is a shearing wave propagation speed in the bone in a direction forming the angle $\theta$ with the first direction.

**15.** Method according to one of claims 1 to 7, wherein, jointly with the speed ($V_{os1}$) of sound in bone in the first direction, an anisotropy parameter of the bone ($\beta$) is determined (708) able to be used by a predetermined function in combination with the speed of sound in bone in the first determined direction to calculate a speed of sound in bone in any direction, said joint determining comprising the sub-steps of:

- for several pairs of second and fourth predetermined candidate values, construction (800) of a second image showing the cortical bone tissue of the bone and the endosteum of the bone, from first echo signals, the speed of sound in non-bone biological tissue in the first determined direction, and the first demarcation curve, under the hypothesis that the speed of sound in bone in the first direction is equal to the second candidate value, and under the hypothesis that the anisotropy parameter of the bone ($\beta$) is equal to the fourth candidate value,
- for each second image, calculating (802) at least one second metric indicative of a focus quality of the cortical bone tissue of the bone and/or of the endosteum of the bone in the second image,
- joint selecting (804) of a second candidate value as the speed of sound in bone in the first direction, and of a fourth candidate value as anisotropy parameter of the bone ($\beta$), the selecting being implemented according to the second metrics.

# FIG. 1

# FIG. 2

EP 3 654 847 B1

## FIG. 3

Emission d'ondes ultrasonores de compression radiales vers le corps comprenant du tissu biologique non osseux et un os ⟋100

Réception de premiers signaux d'écho ⟋102

Détermination d'une vitesse $V_{tissu1}$ de propagation radiale d'ondes de compression dans le tissu biologique non osseux ⟋104

Localisation d'une courbe de démarcation entre le tissu biologique non osseux et l'os ⟋106

Détermination d'une vitesse $V_{os1}$ de propagation radiale dans l'os ⟋108

## FIG. 4

Construction de premières images à partir des premiers signaux d'écho et à l'aide de premières valeurs candidates pour $V_{tissu1}$ ⟋200

Pour chaque première image, calcul d'une première métrique de qualité de mise au point dans la première image ⟋202

Sélection d'une des premières valeurs candidates en tant que vitesse $V_{tissu1}$ en fonction des premières métriques calculées ⟋204

## FIG. 5a

## FIG. 5b

## FIG. 6

Construction de deuxièmes images à partir des premiers signaux d'écho et à l'aide de premières valeurs candidates pour $V_{OS1}$, et à partir de $V_{tissu1}$ et de la première courbe de démarcation — 300

Pour chaque deuxième image, calcul d'une deuxième métrique de qualité de mise au point dans la deuxième image — 302

Sélection d'une des deuxièmes valeurs candidates en tant que vitesse $V_{OS1}$ en fonction des deuxièmes métriques calculées — 304

**FIG. 7**

# FIG. 8

Qualité de mise au point

$V_{radial}$ (m/s)

Énergie spectrale latérale
Énergie spatiale 2D
Netteté de Brenner
Netteté de Tenenbaum
NormVariance

EP 3 654 847 B1

## FIG. 9

| | |
|---|---|
| Emission d'ondes ultrasonores vers le corps comprenant du tissu biologique non osseux et un os | 400 |
| Réception de deuxièmes signaux d'écho | 402 |
| Détermination d'une vitesse $V_{tissu2}$ de propagation axiale d'ondes de compression dans le tissu biologique non osseux | 404 |
| Localisation d'une deuxième courbe de démarcation entre le tissu biologique non osseux et l'os | 408 |
| Détermination éventuelle d'une vitesse $V_{os2}$ de propagation axiale d'ondes de compression dans l'os | 410 |
| Détermination d'un paramètre $\beta$ d'anisotropie de l'os | 412 |

## FIG. 10

Construction de troisièmes images à partir des deuxièmes signaux d'écho et à l'aide de troisièmes valeurs candidates pour $V_{tissu2}$ — 500

Pour chaque troisième image, calcul d'une troisième métrique de qualité de mise au point dans la troisième image — 502

Sélection d'une des troisièmes valeurs candidates en tant que vitesse $V_{tissu2}$ en fonction des troisièmes métriques calculées — 504

## FIG. 11

Construction de quatrièmes images à partir des deuxièmes signaux d'écho et à l'aide de quatrièmes valeurs candidates pour le paramètre $\beta$, et à partir de $V_{tissu2}$, éventuellement de $V_{os2}$ et de la deuxième courbe de démarcation — 600

Pour chaque image, calcul d'une deuxième métrique de qualité de mise au point dans la deuxième image — 602

Sélection d'une des quatrièmes valeurs candidates en tant que paramètre $\beta$ en fonction des quatrièmes métriques calculées — 604

FIG. 12

Qualité de mise au point

Paramètre d'anisotropie $\beta$

—·—·—·— Énergie spectrale latérale
················· Énergie spatiale 2D
▪▪▪▪▪▪▪▪▪▪▪▪▪ Netteté de Brenner
————— Netteté de Tenenbaum
————— NormVariance

EP 3 654 847 B1

## FIG. 13

Emission d'ondes ultrasonores de compression axiales vers le corps comprenant du tissu biologique non osseux et un os — 700

Réception de deuxièmes signaux d'écho — 702

Détermination d'une vitesse $V_{tissu2}$ de propagation axiale d'ondes de compression dans le tissu biologique non osseux — 704

Localisation d'une courbe de démarcation entre le tissu biologique non osseux et l'os — 706

Détermination d'une vitesse $V_{OS1}$ de propagation radiale dans l'os et conjointement d'un paramètre d'anisotropie $\beta$ — 708

## FIG. 14

Construction d'images à partir des signaux d'écho et à l'aide de valeurs candidates pour $V_{OS1}$ et de valeurs candidates pour le paramètre d'anisotropie $\beta$ — 800

Pour chaque image, calcul d'une métrique de qualité de mise au point dans l'image — 802

Sélection d'une paire de valeurs candidates pour obtenir la vitesse $V_{OS1}$ et le paramètre $\beta$, en fonction des métriques calculées — 804

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2005004457 A

- US 5038787 A

**Littérature non-brevet citée dans la description**

- **D. NAPOLITANO ; C.CHOU ; G. MCLAUGHLIN et al.** *Sound Speed Correction in ultrasound image,* 2006

- **B. TREEBY ; T. VARSLOT ; E. ZHANG et al.** *Automatic sound speed selection in photoacoustic image reconstruction using an autofocus approach,* 2011